# EUROPÄISCHE PATENTSCHRIFT

(11) **EP 1 915 386 B1**
(45) Veröffentlichungstag und Bekanntmachung des Hinweises auf die Patenterteilung: **08.06.2011**
(21) Anmeldenummer: 06776358.1
(22) Anmeldetag: 24.07.2006
(51) Int. Cl.: C07F 15/00, C07D 213/50, C07D 221/02, C07D 221/06, C07D 471/04, H01L 51/00

(54) **METALLKOMPLEXE**
METAL COMPLEX
COMPLEXES METALLIQUES

(30) Priorität: 18.08.2005 DE 102005039064
(43) Veröffentlichungstag der Anmeldung: 30.04.2008
(73) Patentinhaber: Merck Patent GmbH, 64293 Darmstadt (DE)
(72) Erfinder: SCHWAIGER, Jochen, 60313 Frankfurt (DE)
(86) Internationale Anmeldenummer: PCT/EP2006/007246
(87) Internationale Veröffentlichungsnummer: WO 2007/019942

(56) Entgegenhaltungen:
- EP-A- 1 211 257
- FUCHITA, YOSHIO ET AL: "Synthesis and characterization of the six-membered cyclopalladated complexes of 2-benzylbenzothiazole" INORGANICA CHIMICA ACTA , 239(1-2), 125-32 CODEN: ICHAA3; ISSN: 0020-1693, 1995, XP002397430
- EL-DISSOUKY, ALI ET AL: "Metal chelates of heterocyclic nitrogen-containing ketones. XIV. Metal ion, anion and substituent effects on the enolization of mono-keto compounds" INORGANICA CHIMICA ACTA , 87(2), 213-22 CODEN: ICHAA3; ISSN: 0020-1693, 1984, XP002397431
- DATABASE CA [Online] CHEMICAL ABSTRACTS SERVICE, COLUMBUS, OHIO, US; CANTY, ALLAN J. ET AL: "Synthetic and structural studies of binuclear organopalladium(II) complexes including a bis(pyridin-2-yl)phenylmethyl complex with four- and eight-membered palladocycle rings, trans(N,N)-[Pd(.mu.-Py2PhC- N,N',C')Cl]2.cntdot.0.5CH2Cl2.cntdot.0.5Me 2CO" XP002397435 gefunden im STN Database accession no. 111:78311 & AUSTRALIAN JOURNAL OF CHEMISTRY , 41(5), 651-65 CODEN: AJCHAS; ISSN: 0004-9425, 1988,
- DATABASE CA [Online] CHEMICAL ABSTRACTS SERVICE, COLUMBUS, OHIO, US; EL-DISSOUKY, ALI ET AL: "Metal chelates of heterocyclic nitrogen containing ketones. XIII. Cobalt(II), nickel(II) and palladium(II) complexes of 2-picolyl and 2-lutidyl methyl ketones" XP002397436 gefunden im STN Database accession no. 100:131400 & TRANSITION METAL CHEMISTRY (DORDRECHT, NETHERLANDS) , 9(1), 23-8 CODEN: TMCHDN; ISSN: 0340-4285, 1984,
- DATABASE CA [Online] CHEMICAL ABSTRACTS SERVICE, COLUMBUS, OHIO, US; UHLEMANN, E.: "Picolyl ketones. II. Complex formation of 2-picolyl ketones" XP002397437 gefunden im STN Database accession no. 60:28737 & JOURNAL FUER PRAKTISCHE CHEMIE (LEIPZIG) , 21(5-6), 277-85 CODEN: JPCEAO; ISSN: 0021-8383, 1963,

## Beschreibung

Die vorliegende Erfindung beschreibt neue Materialien, deren Verwendung in organischen Elektrolumineszenzelementen und darauf basierende Displays.

Metallorganische Verbindungen, speziell Ir- und Pt-Verbindungen, finden in einer Reihe verschiedenartiger Anwendungen, die im weitesten Sinne der Elektronikindustrie zugerechnet werden können, Einsatz als funktionelle Materialien, z. B. in organischen Elektrolumineszenzvorrichtungen. Der allgemeine Aufbau solcher Vorrichtungen ist beispiels-weise in US 4,539,507 und US 5,151,629 beschrieben. Eine Entwicklung, die sich in den letzten Jahren abzeichnet, ist der Einsatz metallorganischer Komplexe, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Ob sich diese Entwicklung durchsetzen wird, hängt davon ab, ob entsprechende Device-Kompositionen gefunden werden, die diese Vorteile (Triplett-Emission = Phosphoreszenz gegenüber Singulett-Emission = Fluoreszenz) auch in den OLEDs umsetzen können. Als wesentliche Bedingungen sind hier insbesondere eine hohe operative Lebensdauer und eine hohe thermische Stabilität der Komplexe zu nennen.

Allerdings gibt es bei OLEDs, die Triplettemission zeigen, immer noch erhebliche Probleme, die einer dringenden Verbesserung bedürfen. Dies gilt insbesondere auch für den Triplettemitter selbst. Die meisten in der Literatur bekannten Komplexe enthalten Liganden, basierend auf Phenylpyridin oder verwandten Strukturen, die an Iridium oder Platin koordinieren (z. B. WO 02/068435, WO 04/026886 und EP1211257).

Verbindungen dieses Typs weisen in der Praxis einige entscheidende Schwachpunkte auf, welche einer Verbesserung bedürfen:
1. Ein entscheidender Mangel ist die geringe thermische Stabilität der oben beschriebenen Verbindungen. So kann zum Beispiel der homoleptische Komplex *fac*-Tris(1-phenyl-isochinolin-C²,N)iridium(III) (Ir(piq)₃) nicht unzersetzt sublimiert werden. Selbst unter typischen Hochvakuumbedingungen (p < 10⁻⁷ mbar) beobachtet man eine erhebliche Zersetzung dieser Verbindung, wobei neben einer iridiumhaltigen Asche, die ca. 30 Gew.% der eingesetzten Menge an Ir(piq)₃ ausmacht, die Freisetzung von 1-Phenylisochinolin und anderen niedermolekularen Verbindungen nachgewiesen werden kann. Diese thermische Zersetzung führt zu einer wenig reproduzierbaren Device-Charakteristik, wobei die Lebensdauer besonders negativ betroffen ist. Auch bei der Reinigung der Metallkomplexe durch Sublimation ist es erforderlich, temperaturstabilere Komplexe zur Verfügung zu haben, da die Zersetzung zu großen Verlusten der Komplexe führt.
2. Die operative Lebensdauer ist allgemein zu gering, was bislang noch der Einführung von phosphoreszierenden OLEDs in hochwertigen und langlebigen Vorrichtungen entgegensteht.
3. Die Komplexe besitzen häufig nur eine geringe Löslichkeit in organischen Lösemitteln, was eine effiziente Reinigung durch Umkristallisation oder Chromatographie stark erschwert oder verhindert. Dies gilt insbesondere für die Reinigung größerer Mengen, wie sie in der Displayfertigung benötigt werden. Gerade auch die bromierten Komplexe, die beispielsweise zur Herstellung von Polymeren verwendet werden können, zeigen nur eine geringe Löslichkeit und sind daher bei der Polymerisation schwer-zu verarbeiten.
4. Die Liganden für Systeme gemäß dem Stand der Technik, insbesondere für rot emittierende Komplexe, sind nur in aufwändigen mehrstufigen Verfahren zugänglich.

Es besteht daher der Bedarf an Verbindungen, die die oben genannten Schwachpunkte nicht aufweisen, den bekannten Metallkomplexen jedoch in Bezug auf Effizienz und Emissionsfarbe mindestens gleichwertig sind. In US 2004/241493 wird ein 5-Ring-Chelat einer Iminverbindung mit Iridium als weißer bis weißlicher Emitter beschrieben. Es ist nicht offensichtlich, wie mit diesen Komplexen andere Farben erzielt werden können.

Überraschend wurde gefunden, dass bestimmte neue Verbindungen, die statt des oben beschriebenen Fünfring-Chelates ein Sechsring-Chelat mit einer Iminverbindung mit Iridium verwenden, verbesserte Eigenschaften als Triplettemitter in OLEDs besitzen. Zur Erläuterung sind im Folgenden ein Iridium-Fünfring-Chelat und ein Iridium-Sechsring-Chelat abgebildet, wobei D ein koordinierendes Atom, beispielsweise Stickstoff, darstellt und C wie üblich für Kohlenstoff steht:

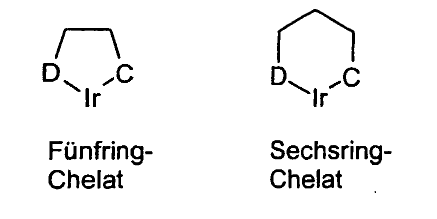

Metallkomplexe, die Sechsring- und Siebenring-Chelate für die Verwendung in OLEDs aufweisen, sind teilweise bereits in der Literatur beschrieben:
In EP 1211257 werden Metallkomplexe beschrieben, die zwischen dem Phenyl- und dem Pyridinring des Liganden eine nicht-konjugierte Einheit X enthalten, beispielsweise O, S, CR₂, etc., wodurch Sechsring-Chelat-Komplexe mit nicht durchgängig konjugierten Ligandensystemen entstehen. Diese Komplexe zeigen blaue bis orange-rote Emission, wie den Beispielen der oben genannten Anmeldung zu entnehmen ist, sind aber offensichtlich nicht für die Erzeugung tiefroter Emission geeignet, was möglicherweise durch die fehlende Konjugation des Liganden zu begründen ist.
In JP 2003/342284 werden ähnliche Sechsring-Chelat-Komplexe beschrieben, wobei die Einheit X Teil eines größeren Ringsystems ist. Insbesondere ist X der Stickstoff eines Carbazol-Systems oder ein Kohlenstoff in 9-Position eines Fluorens. Hierbei entstehen wiederum Systeme, deren Liganden nicht konjugiert sind.
In JP 2004/111193 werden konjugierte und nicht-konjugierte Siebenring-Chelat-Komplexe beschrieben.

### Gegenstand der vorliegenden Erfindung sind Verbindungen gemäß Formel (1)

M(L)ₙ(L')ₘ(L")ₒ Formel (1)

enthaltend eine Teilstruktur M(L)ₙ gemäß Formel (2), 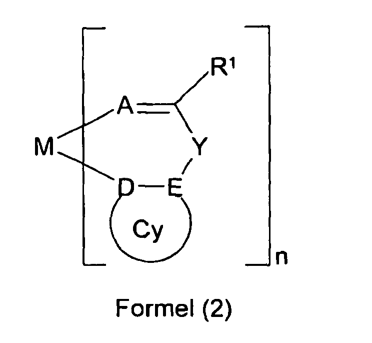 wobei für die verwendeten Symbole und Indizes gilt:
- M: ist ein Übergangsmetall;
- A: ist gleich oder verschieden bei jedem Auftreten NR;
- D: ist ein sp²-hybridisiertes Kohlenstoffatom, das an M bindet;
- E: ist gleich oder verschieden bei jedem Auftreten ein sp²-hybridisiertes Kohlenstoff- oder Stickstoffatom;
- Cy: ist gleich oder verschieden bei jedem Auftreten ein Homo- oder Heterocyclus, der über ein sp²-hybridisiertes Kohlenstoffatom an M bindet und an den gegebenenfalls eine oder mehrere Gruppen R gebunden sind;
- Y: ist gleich oder verschieden bei jedem Auftreten CR₂, C(=O), C(=NR), C(=N-NR₂), C(=CR₂), SiR₂, O, S, S(=O), S(=O)₂, Se, NR, PR, P(=O)R, AsR, As(=O)R oder BR;
- R, R¹: ist gleich oder verschieden bei jedem Auftreten H, F, CI, Br, I, CN, B(OH)₂, B(OR²)₂, NO₂, eine geradkettige Alkyl- oder Alkoxygruppe mit 1 bis 40 C-Atomen oder eine verzweigte oder cyclische Alkyl- oder Alkoxygruppe mit 3 bis 40 C-Atomen, wobei eine oder mehrere nicht benachbarte CH₂-Gruppen durch -R²C=CR²-, C≡C-, Si(R²)_{2,} Ge(R²)₂, Sn(R²)₂, -O-, -S-, -NR²-, -(C=O)-, -(C=NR²)-, -P=O(R²)- oder -CONR²- ersetzt sein können und wobei ein oder mehrere H-Atome durch F, Cl, Br, I ersetzt sein können, oder ein aromatisches oder heteroaromatisches Ringsystem bzw. eine Aryloxy- oder Heteroaryloxy- gruppe mit 5 bis 40 C-Atomen, welches durch einen oder mehrere nicht-aromatische Reste R substituiert sein kann; dabei kann R auch mit Cy ein weiteres aliphatisches oder aromatisches Ringsystem bilden; dabei können die Substituenten R, welche an Y binden, mit dem Cyclus Cy und/oder mit dem Rest R1 einen Fünfring oder einen Sechsring bilden;
- R²: ist gleich oder verschieden bei jedem Auftreten H oder ein aliphatischer oder aromatischer Kohlenwasserstoffrest mit 1 bis 20 C-Atomen, bei dem einzelne H-Atome auch durch F ersetzt sein können; dabei können auch zwei oder mehrere Reste R² miteinander ein Ringsystem bilden;
- n: ist 1, 2 oder 3;
dabei sind die Liganden L' und L" in Formel (1) monoanionische, zweizähnig chelatisierende Liganden; m und o sind gleich oder verschieden bei jedem Auftreten 0, 1 oder 2; dabei gilt, dass n + m + o = 2 für quadratisch-planar koordinierte Metalle, beispielsweise Platin und Palladium, und n + m + o = 3 für oktaedrisch koordinierte Metalle, beispielsweise Iridium, ist.

Unter Hybridisierung wird die Linearkombination von Atomorbitalen verstanden. So entstehen durch Linearkombination von einem 2s- und zwei 2p-Orbitalen drei äquivalente sp²-Hybridorbitale, die einen Winkel von 120° miteinander bilden. Das verbleibende p-Orbital ist zur Ausbildung einer π-Bindung, beispielsweise in einem aromatischen System, befähigt.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen System mit 5-40 aromatischen Ringatomen, welches noch jeweils mit den oben genannten Resten R¹ substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Tetracen, Pentacen, Benzpyren, Biphenyl, Biphenylen, Terphenyl, Terphenylen, Fluoren, Spirobifluoren, Truxen, Isotruxen, Dihydrophenanthren, Dihydropyren, Tetrahydropyren, cis- oder trans-Indenofluoren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

### Bevorzugt ist Cy ein aromatisches bzw. heteroaromatisches System.

Bevorzugt sind Verbindungen gemäß Formel (1), enthaltend eine Teilstruktur M(L)ₙ gemäß Formel (2a), 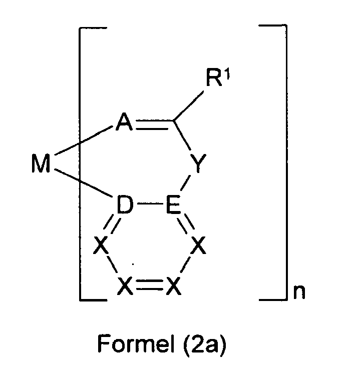 wobei M, A, Y, R, R¹, R², L', L", n, m und o dieselbe Bedeutung haben, wie oben beschrieben, und für die weiteren Symbole gilt:
- D: ist ein sp²-hybridisiertes Kohlenstoffatom, das an M bindet,;
- X: ist gleich oder verschieden bei jedem Auftreten CR¹, N oder P; oder
- (X-X) bzw. (X=X): (also zwei benachbarte X) steht für NR¹, S oder O; oder
- (X-X) bzw. (X=X): (also zwei benachbarte X) steht für CR¹, N oder P, falls das Symbol E für N steht; mit der Maßgabe, dass Cy einen 5- oder 6-Ring darstellt;
- E: ist gleich oder verschieden bei jedem Auftreten C oder N mit der Maßgabe, dass, falls das Symbol E für N steht, genau eine Einheit X-X (also zwei benachbarte X) in Cy gleich CR¹, N oder P ist.

Eine besonders bevorzugte Ausführungsform der vorliegenden Erfindung sind Verbindungen der Formel (1a).

M(L)ₙ(L')ₘ(L")ₒ Formel (1a)

enthaltend mindestens eine Teilstruktur M(L)ₙ der Formel (2b), 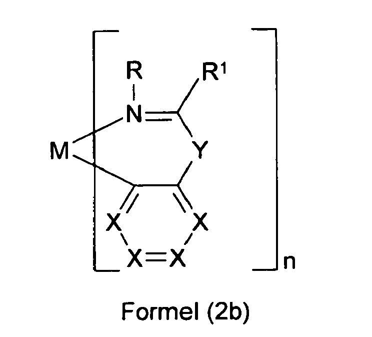 und gegebenenfalls enthaltend eine Teilstruktur M(L')ₘ der Formel (3), 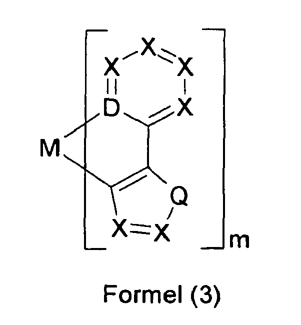 wobei M, Y, D, R, R¹, R², L", n, m und o dieselbe Bedeutung haben, wie oben beschrieben, und weiterhin gilt:
- X: ist gleich oder verschieden bei jedem Auftreten CR¹ oder N; oder
- (X-X) bzw. (X=X): (also zwei benachbarte X) steht für NR¹, S oder O; mit der Maßgabe, dass Cy einen Fünf- oder einen Sechsring darstellt
- Q: ist gleich oder verschieden bei jedem Auftreten -CR¹=CR¹-, -N=CR¹-, -N=N-, NR¹, O oder S.

Bevorzugt sind Verbindungen gemäß Formel (1) bzw. Formel (1a), in denen das Symbol Y gleich oder verschieden CR₂, C(=O), C(=CR₂), O, S, NR, PR, P(=O)R oder BR ist. Besonders bevorzugt sind Verbindungen gemäß Formel (1) bzw. Formel (1a), in denen das Symbol Y gleich oder verschieden CR₂, O, S, NR oder P(=O)R ist. Ganz besonders bevorzugt sind Verbindungen gemäß Formel (1) bzw. Formel (1a), in denen das Symbol Y gleich CR₂ oder NR ist.

Erfindungsgemäße monoanionische, zweizähnige Liganden L" sind 1,3-Diketonate abgeleitet von 1,3-Diketonen, wie z. B. Acetylaceton, Benzoylaceton, 1,5-Diphenylacetylaceton, Bis(1,1,1-trifluoracetyl)methan, 3-Ketonate abgeleitet von 3-Ketoestern, wie z. B. Acetessigsäureethylester, Carboxylate abgeleitet von Aminocarbonsäuren, wie z. B. Pyridin-2-carbonsäure, Chinolin-2-carbonsäure, Glycin, N,N-Dimethylglycin, Alanin, N,N-Dimethylalanin, Salicyliminate abgeleitet von Salicyliminen, wie z. B. Methylsalicylimin, Ethylsalicylimin, Phenylsalicylimin, sowie Borate Stickstoff-haltiger Heterocyclen, wie z. B. Tetrakis(1-imidazolyl)borat und Tetrakis(1-pyrazolyl)borat.

In einer besonders bevorzugten Ausführungsform der Erfindung handelt es sich um steife Systeme gemäß den Formeln (2d) bis (2f), in denen die Substituenten R von Y mit dem Cyclus Cy und/oder dem Rest R¹ einen Fünfring oder einen Sechsring bilden, 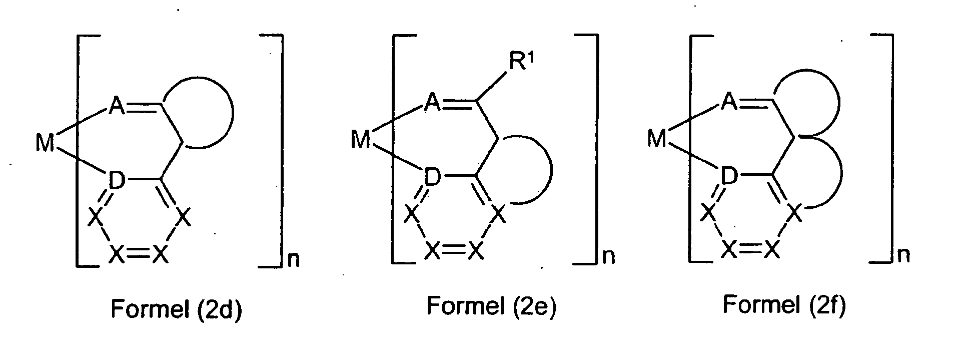 wobei die Symbole und Indizes dieselbe Bedeutung haben, wie oben beschrieben.

Ganz besonders bevorzugt sind Verbindungen gemäß Formel (1) bzw. Formel (1a), enthaltend eine Teilstruktur M(L)ₙ gemäß Formel (2g), gemäß Formel (2h) oder gemäß Formel (2i), 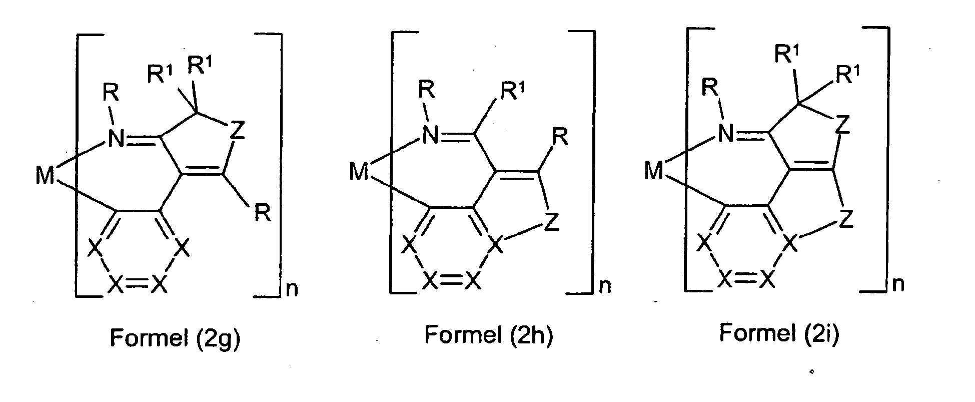 wobei M, X, R, R¹, R² und n dieselbe Bedeutung haben, wie oben beschrieben, und für das Symbol Z gilt:
- Z: ist bei jedem Auftreten gleich oder verschieden eine bivalente Gruppe -C(R¹)₂-, -C(=O)-, -C[=C(R¹)₂]-, -C(R¹)₂-C(R¹)₂-, -C(R¹)₂-C(R¹)₂-C(R¹)₂-, -C(R¹)₂-C-C(R¹)₂-, -C(R¹)₂-N(R¹)-, -C(R¹)=C(R¹)-, -C(R¹)=N-, -O-, -S-, -N(R¹)-, -P(R¹)-, -P(=O)(R¹)- oder -B(R¹)-_{.}

In einer weiteren bevorzugten Ausführungsform der Erfindung ist der Ligand so aufgebaut und substituiert, dass sich daraus bei Koordination an ein Metall kein Fünfring-Chelat bilden kann.

Bevorzugt sind weiterhin Verbindungen gemäß Formel (1) bzw. Formel (1 a), bei denen für das Symbol M = Rh, Ir, Pd oder Pt gilt; besonders bevorzugt gilt M = Ir oder Pt, insbesondere Ir.

Bevorzugt sind weiterhin Verbindungen gemäß Formel (1) bzw. Formel (1a), bei denen für das Symbol n = 2 oder 3 gilt. Besonders bevorzugt sind Verbindungen, bei denen für das Symbol o = 0 gilt. Ganz besonders bevorzugt sind Verbindungen, bei denen für die Symbole m = o = 0 gilt. Dabei gilt insbesondere, dass n = 2 und m = o = 0 für Palladium- und Platin-Komplexe und n = 3 und m = o = 0 für Rhodium- und IridiumKomplexe bevorzugt ist.

Bevorzugt sind weiterhin Verbindungen gemäß Formel (1) bzw. Formel (1a), bei denen das Symbol X für CR¹ steht.

Bevorzugt sind die erfindungsgemäßen Verbindungen gemäß Formel (1) bzw. Formel (1a), bei denen das Symbol Z in Formel (2g), (2h) bzw. (2i) für eine bivalente Gruppe -C(R¹)₂-, -C(=O)-, -C(R¹)₂-C(R¹)₂-, -C(R¹)₂-N(R¹)-, -C(R¹)=C(R¹)-, -C(R¹)=N-, -O-, -S- oder -N(R¹)- steht. Besonders bevorzugt steht das Symbol Z für -C(R¹)₂-, -C(R¹)₂-C(R¹)₂-, -C(R¹)=C(R¹)-, -S- oder -N(R¹)-.

Die entsprechenden Liganden, die Teilstrukturen gemäß Formel (2) bzw. Formel (2a) bis (2i) erzeugen, bzw. auch die Liganden L' und L" können nach gängigen organisch-chemischen Verfahren dargestellt werden, wie sie dem Fachmann der organischen Synthese geläufig sind. Die Vorstufen der Iminsynthese sind häufig kommerziell erhältliche β-Ketoverbindungen, die mit Aminen unter Wasserabspaltung zu Iminen reagieren. Diese Synthese ist deutlich einfacher als die üblichen Ligandensynthesen gemäß dem Stand der Technik.

Die erfindungsgemäßen Metallkomplexe sind prinzipiell durch verschiedene Verfahren darstellbar; es haben sich jedoch die im Folgenden beschriebenen Verfahren als besonders gut geeignet herausgestellt.

Daher ist ein weiterer Gegenstand der vorliegenden Erfindung ein Verfahren zur Herstellung der erfindungsgemäßen Metallkomplexe durch Umsetzung der entsprechenden freien Liganden mit Metallalkoholaten der Formel (4), mit Metallketoketonaten der Formel (5) oder ein- oder mehr-kernigen Metallhalogeniden der Formel (6), (7) oder (8), 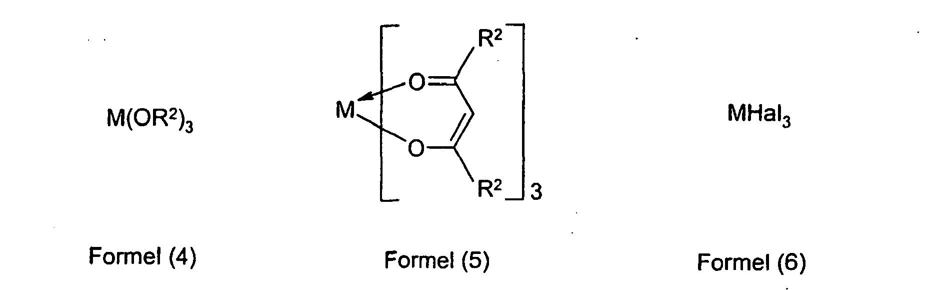 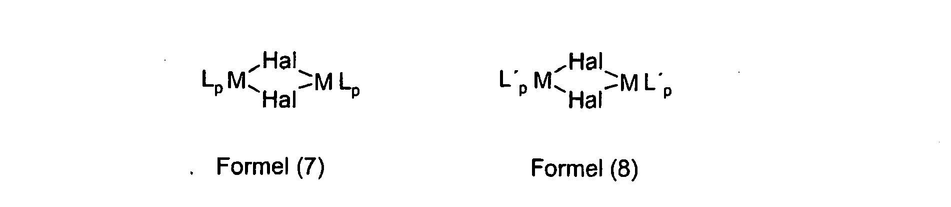 wobei die Symbole M und R² die oben angegebenen Bedeutungen haben, p = 1 oder 2 und Hal = F, Cl, Br oder I ist.

Es können ebenfalls Metallverbindungen, bevorzugt Rhodium- und Iridiumverbindungen, die sowohl Alkoholat- und/oder Halogenid- und/oder Hydroxywie auch Ketoketonatreste tragen, verwendet werden. Diese Verbindungen können auch geladen sein. Entsprechende Iridiumverbindungen, die als Edukte besonders geeignet sind, sind in WO 04/085449 offenbart.

Die Synthese der Komplexe wird bevorzugt durchgeführt, wie in WO 02/060910 und in WO 04/085449 beschrieben. Heteroleptische Komplexe können beispielsweise auch gemäß WO 05/042548 synthetisiert werden.

Durch diese Verfahren lassen sich die erfindungsgemäßen Verbindungen gemäß Formel (1) in hoher Reinheit, bevorzugt mehr als 99 % (bestimmt mittels ¹H-NMR und/oder HPLC) erhalten.

Mit den hier erläuterten Synthesemethoden lassen sich unter anderem die im Folgenden dargestellten Strukturen (1) bis (28) für die Verbindungen gemäß Formel (1) herstellen, die noch durch Substituenten R¹ substituiert sein können. Diese Substituenten sind hier in den meisten Fällen der Übersichtlichkeit halber nicht abgebildet.

| | | |
|---|---|---|
| 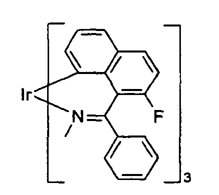 | 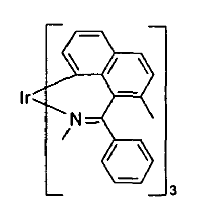 | |
| (1) | (2) | |
| 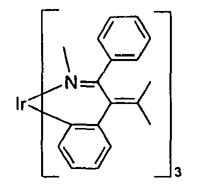 | 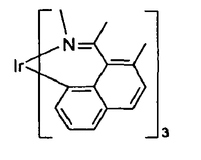 | 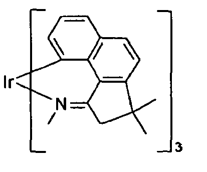 |
| (4) | (5) | (6) |
| 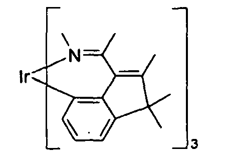 | 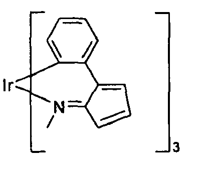 | 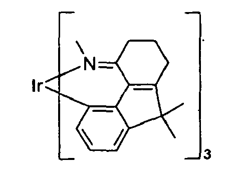 |
| (7) | (8) | (9) |
| 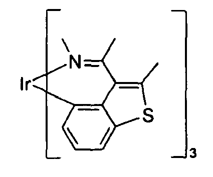 | 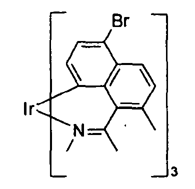 | 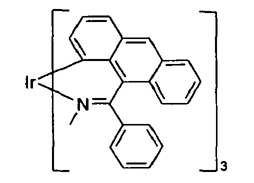 |
| (10) | (11) | (12) |
| 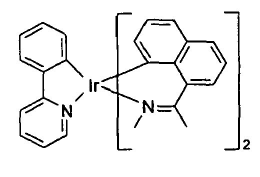 | 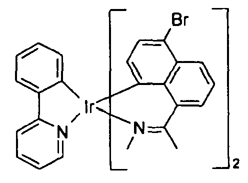 | 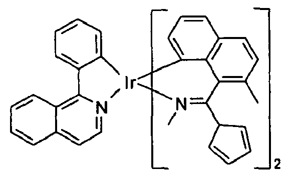 |
| (13) | (14) | (15) |
| 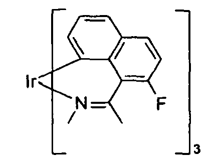 | | |
| (16) | | |
| 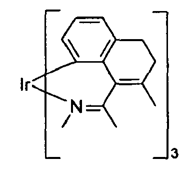 | 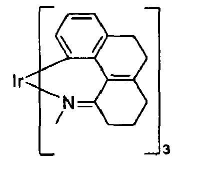 | 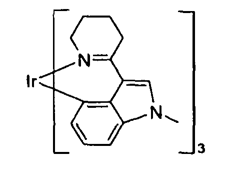 |
| (19) | (20) | (21) |
| 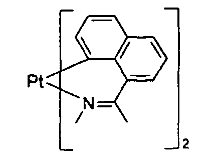 | 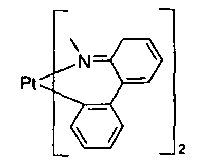 | 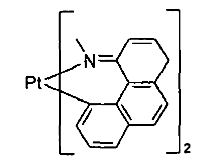 |
| (22) | (23) | (24) |
| 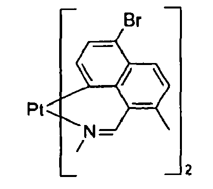 | 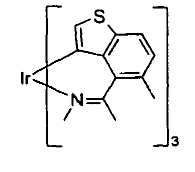 | 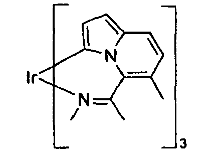 |
| (25) | (26) | (27) |
| 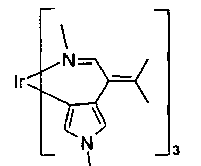 | | |
| (28) | | |

Die oben beschriebenen erfindungsgemäßen Verbindungen, z. B. die Verbindungen (11), (14) und (25), können auch als Comonomere zur Erzeugung entsprechender konjugierter, teilkonjugierter oder nichtkonjugierter Oligomere, Polymere oder Dendrimere Verwendung finden. Die Polymerisation erfolgt dabei bevorzugt über die Bromfunktionalität. Weitere Wiederholeinheiten der Polymere sind bevorzugt ausgewählt aus der Gruppe bestehend aus Fluorenen (z. B. gemäß EP 842208 oder

WO 00/22026), Spirobifluorenen (z. B. gemäß EP 707020 oder EP 894107), Dihydrophenanthrenen (z. B. gemäß WO 05/014689), Indenofluorenen (z. B. gemäß WO 04/041901 und WO 04/113412), Phenanthrenen (z. B. gemäß WO 05/104264), para-Phenylenen (z. B. gemäß WO 92/18552), Carbazolen (z. B. gemäß WO 04/070772 oder WO 04/113468), Ketonen (z. B. gemäß WO 05/040302), Silanen (z. B. gemäß WO 05/111113), Triarylaminen oder Thiophenen (z. B. gemäß EP 1028136) oder auch mehrere verschiedene dieser Einheiten. Dabei können sie entweder in die Seitenkette oder in die Hauptkette des Polymers eingebaut werden oder können auch Verzweigungspunkte der Polymerketten (z. B. gemäß WO 06/003000) darstellen.

Weiterer Gegenstand der Erfindung sind somit konjugierte, teilkonjugierte oder nicht-konjugierte Oligomere, Polymere oder Dendrimere, enthaltend eine oder mehrere der Verbindungen gemäß Formel (1) bzw. Formel (1a), wobei mindestens einer der oben definierten Reste R bzw. R¹, bevorzugt R¹, eine Bindung zum Polymer oder Dendrimer darstellt. Für Einheiten gemäß Formel (1) bzw. Formel (1a) gelten in Polymeren und Dendrimeren dieselben Bevorzugungen, wie oben bereits beschrieben.

Die oben genannten Oligomere, Polymere, Copolymere und Dendrimere zeichnen sich durch ihre gute Löslichkeit in organischen Lösemitteln und hohe Effizienz und Stabilität in organischen elektrolumineszierenden Vorrichtungen aus.

Weiterhin können die erfindungsgemäßen Verbindungen gemäß Formel (1), insbesondere solche, die durch Halogene funktionalisiert sind, auch durch gängige Reaktionstypen weiter funktionalisiert werden und so zu erweiterten Verbindungen gemäß Formel (1) umgesetzt werden. Hier ist als Beispiel die Funktionalisierung mit Arylboronsäuren gemäß Suzuki oder mit Aminen gemäß Hartwig-Buchwald zu nennen.

Die erfindungsgemäßen Verbindungen, Oligomere, Polymere, Dendrimere oder erweiterten Verbindungen gemäß Formel (1) finden Verwendung als aktive Komponenten in elektronischen Bauteilen, wie z. B. organischen Leuchtdioden (OLEDs), organischen integrierten Schaltungen (O-ICs), organischen Feld-Effekt-Transistoren (O-FETs), organischen Dünnfilmtransistoren (O-TFTs), organischen Feld-Quench-Devices (O-FQDs), organischen lichtemittierenden Transistoren (O-LETs), lichtemittierenden elektrochemischen Zellen (LECs), organischen Solarzellen (O-SCs) oder organischen Laserdioden (O-Laser).

Gegenstand der vorliegenden Erfindung ist also weiterhin die Verwendung der erfindungsgemäßen Verbindungen gemäß Formel (1), der erfindungsgemäßen Oligomere, Polymere und Dendrimere und entsprechender erweiterter Verbindungen gemäß Formel (1) als aktive Komponente in elektronischen Bauteilen, insbesondere als emittierende Verbindung.

Weiterer Gegenstand der Erfindung sind elektronische Bauteile, insbesondere organische und polymere Leuchtdioden (OLEDs, PLEDs), organische Feld-Effekt-Transistoren (O-FETs), organische Dünnfilmtransistoren (O-TFTs), organische integrierte Schaltungen (O-ICs), organische Feld-Quench-Devices (O-FQDs), organische lichtemittierende Transistoren (O-LETs), lichtemittierende elektrochemische Zellen (LECs), organische Solarzellen (O-SCs) und organische Laserdioden (O-Laser), enthaltend eine oder mehrere erfindungsgemäße Verbindungen gemäß Formel (1), erfindungsgemäße Oligomere, Polymere und Dendrimere und entsprechende erweiterte Verbindungen gemäß Formel (1), insbesondere als emittierende Verbindung.

Bevorzugt werden die erfindungsgemäßen Verbindungen als emittierende Verbindungen in einer emittierenden Schicht in einer organischen oder polymeren Leuchtdiode eingesetzt. Insbesondere wenn es sich um niedermolekulare erfindungsgemäße Verbindungen handelt, werden diese üblicherweise zusammen mit einem Matrix-Material eingesetzt. Dabei kann das Matrixmaterial sowohl niedermolekular, wie auch oligomer oder polymer sein.

Bevorzugte Matrixmaterialien sind solche auf Basis von Carbazolen, beispielsweise CBP (Bis(carbazolyl)biphenyl), aber auch andere Materialien enthaltend Carbazol oder Carbazol-Derivate, z. B. gemäß WO 00/057676, EP 01/202358 und WO 02/074015. Bevorzugt sind weiterhin Ketone und Imine, wie beispielsweise in WO 04/093207 beschrieben, insbesondere solche, basierend auf Spirobifluoren, und Phosphinoxide, Phosphinselenide, Phosphazene, Sulfoxide und Sulfone, wie beispielsweise in WO 05/003253 beschrieben, insbesondere solche, basierend auf Spirobifluoren. Weiterhin bevorzugt sind Silane, polypodale Metallkomplexe, z. B. gemäß WO 04/081017 und Oligophenylene basierend auf Spirobifluorenen, z. B. gemäß EP 676461 und WO 99/40051. Besonders bevorzugte Matrixmaterialien sind Ketone, Phosphinoxide, Sulfoxide und Sulfone. Ganz besonders bevorzugt sind Ketone und Phosphinoxide.

Die erfindungsgemäßen Verbindungen weisen die folgenden Vorteile gegenüber Verbindungen gemäß dem Stand der Technik auf:
1. Die erfindungsgemäßen Verbindungen zeichnen sich durch eine hohe Temperaturstabilität aus. So können die niedermolekularen Verbindungen im Hochvakuum unzersetzt verdampft werden, und auch die oligomeren, dendritischen und polymeren Verbindungen sind thermisch sehr stabil, so dass die Vorrichtungen ohne Schaden thermisch behandelt werden können. Diese Eigenschaft ist eine Grundvoraussetzung zur reproduzierbaren Darstellung von OLEDs und wirkt sich insbesondere positiv auf die operative Lebensdauer aus. Weiterhin ist so die ressourcenschonende Nutzung von Verbindungen dieser seltenen Metalle möglich, da die Komplexe bei der Reinigung nahezu verlustfrei sublimiert werden können.
2. Die erfindungsgemäßen Verbindungen zeichnen sich durch eine gute Löslichkeit in organischen Lösungsmitteln aus, was ihre Reinigung durch gängige Verfahren wie Umkristallisation oder Chromatographie erheblich erleichtert. Damit sind die Verbindungen auch aus Lösung durch Beschichtungs- oder Drucktechniken verarbeitbar. Auch bei der üblichen Verarbeitung durch Verdampfen ist diese Eigenschaft von Vorteil, da so die Reinigung der Anlagen bzw. der eingesetzten Schattenmasken erheblich erleichtert wird.
3. Die Synthese der erfindungsgemäßen Verbindungen zeichnet sich durch eine wesentlich vereinfachte Synthese der Liganden aus im Gegensatz zu den komplexen Systemen gemäß dem Stand der Technik. So sind diese Liganden in wenigen Stufen, teilweise sogar in Einstufensynthesen, aus kommerziell erhältlichen Verbindungen zugänglich, wohingegen zur Ligandensynthese gemäß dem Stand der Technik, insbesondere für rot emittierende Systeme, aufwändige Synthesen notwendig sind.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR bezogen werden.

### Beispiel 1: Synthese von Iridiumkomplex Ir1

### a) Synthese des Liganden L1

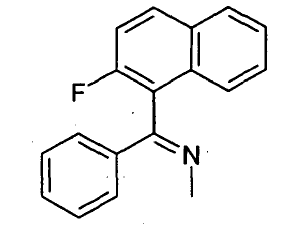

Eine Lösung von 25.1 g (0.272 mol) Phenylmagnesiumbromid in trockenem 100 ml THF wird auf 0 °C gekühlt. Zu dieser Lösung wird eine Lösung aus 46.6 g (0.272 mol) 1-Cyano-2-Fluornaphthalin in 700 ml trockenem THF während 15 min. gegeben. Die Reaktionsmischung wird über Nacht unter Rückfluss erhitzt und dann auf-78 °C gekühlt. Zu der Mischung werden 187.5 ml (0.3 mol) n-Butyllithium (1.6 M Lösung in Hexan) gegeben, und es wird 30 min. gerührt. Dann werden 18.7 ml (0.3 mol) Methyliodid in 200 ml trockenem THF zugegeben. Die Reaktionsmischung wird auf Raumtemperatur erwärmt und 2 h gerührt. Es werden 300 ml Wasser und 500 ml Diethylether zugegeben, und die wässrige Phase wird abgetrennt und dreimal mit Diethylether extrahiert. Die vereinigten organischen Phasen werden mit gesättigter Kochsalzlösung gewaschen und über MgSO₄ getrocknet. Nach Abtrennen des Lösemittels wird das Produkt durch Säulenchromatographie an Silica (Heptan : EtOAc 65 : 35) gereinigt. Ausbeute: 43.3 g (entspricht 60.4 % d. Th.).

### b) Synthese des Chloro-verbrückten Iridiumkomplexdimers

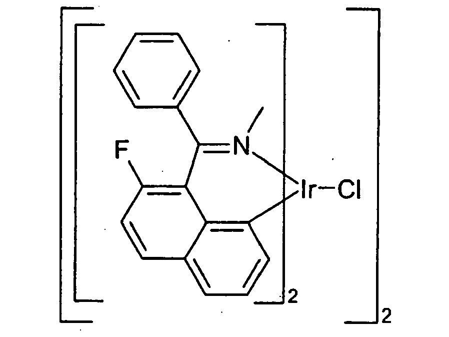

Eine entgaste Lösung aus 8.2 g (17 mmol) Nalr(acac)₂Cl₂ und 21.8 g (83 mmol) des Liganden **L1** in 400 ml Triethylenglycoldimethylether wird 48 h unter Rückfluss erhitzt. Die Suspension wird auf Raumtemperatur gekühlt, der Niederschlag wird abgesaugt, mit Triethylenglycoldimethylether und Ethanol gewaschen und im Vakuum getrocknet. Ausbeute: 9.7 g (73.6 % d. Th.).

### c) Synthese der Komplexes Ir1

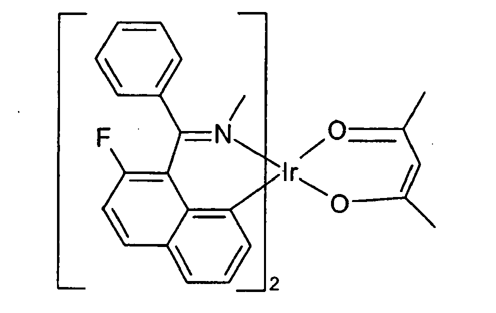

Eine entgaste Mischung aus 2.4 g (1.73 mmol) des Komplexes aus Beispiel 1b). 0.43 g (4.29 mmol) Acetylaceton und 1.95 g (18.4 mmol) Natriumcarbonat in 86 ml Methoxyethanol wird 16 h unter Rückfluss erhitzt. Die Reaktionsmischung wird auf Raumtemperatur abgekühlt und der Niederschlag abgesaugt. Der Feststoff wird mit Ethanol und Hexan gewaschen.

### Beispiel 2: Synthese von Iridiumkomplex Ir2

### a) Synthese der Vorstufe des Liganden L2

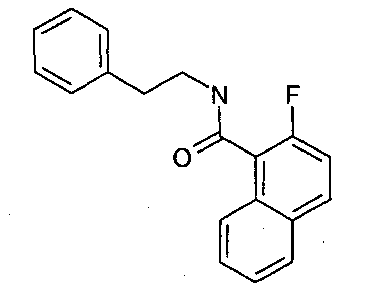

252 ml (2 mol) Phenylethylamin und 230 ml (2.24 mol) Triethylamin werden in 500 ml Dichlormethan gelöst. Zu der Lösung werden bei 0 °C 232 ml (2 mol) 2-Fluornaphthylsäurechlorid (gelöst in 200 ml Dichlormethan) so zugetropft, dass die Temperatur nicht über 40 °C steigt. Es wird über Nacht bei Raumtemperatur gerührt. Der ausgefallene weiße Feststoff wird in 3000 ml Dichlormethan gelöst. Es werden 700 ml 2M NaOH zugegben. Dann wird zweimal mit je 700 ml verdünnter NaOH, dreimal mit je 700 ml 1 M HCl und zweimal mit je 700 ml gesättigter NaHCO₃-Lösung gewaschen. Die organische Phase wird über MgSO₄ getrocknet, und das Lösemittel wird im Vakuum entfernt. Der Feststoff wird mit wenig Dichlormethan nachgewaschen und im Vakuum getrocknet. Ausbeute: 475.6 g (81.1 % d. Th.).

### b) Synthese des Liganden L2

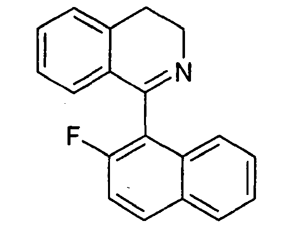

65.1 g (0.222 mol) N-Phenylethyl-2-fluornaphthylamid aus Beispiel 2a) wird bei 90 °C in 375 ml Xylol gelöst. Anschließend werden portionsweise 75.5 g (0.266 mol) P₂O₅ zugegeben, und die Reaktionsmischung wird unter Rückfluss erhitzt. Es werden 140 ml (0.658 mol) POCl₃ zu der heißen Reaktionsmischung getropft, und die Mischung wird weitere 3 h unter Rückfluss erhitzt. Die heiße Reaktionsmischung wird auf Eis gegossen und vorsichtig mit festem NaOH versetzt, bis ein pH von 12 erreicht ist. Das ausgefallene Phosphat wird in 4 L Wasser aufgelöst. Die Phasen werden getrennt, und die wässrige Phase wird dreimal mit je 300 ml Toluol extrahiert. Die vereinigten organischen Phasen werden dreimal mit je 300 ml HCl (pH 1) extrahiert. Die wässrige Phase wir mit 300 ml Toluol versetzt und mit festem NaOH wieder auf pH 12 gebracht. Anschließend wird die wässrige Phase dreimal mit je 150 ml Toluol extrahiert. Die vereinigten organischen Phasen werden über MgSO₄ getrocknet und das Lösemittel im Vakuum entfernt. Ausbeute: 89.7 g (97.1 % d. Th.).

### c) Synthese des Komplexes Ir2

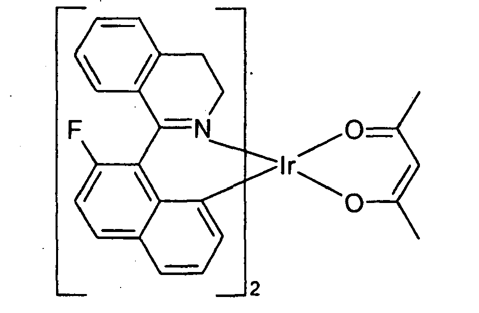

Die Synthese von Ir2 erfolgt in Analogie zu der Synthese aus Beispiel 1 b) und 1c).

### Beispiel 3-5: Herstellung und Charakterisierung von organischen Elektrolumineszenzvorrichtungen mit Verbindungen Ir1 und Ir2

Erfindungsgemäße Elektrolumineszenzvorrichtungen können, wie beispielsweise in WO 05/003253 beschrieben, dargestellt werden. Hier werden die Ergebnisse zweier verschiedener OLEDs gegenübergestellt. Der grundlegende Aufbau, die verwendeten Materialien, der Dotierungsgrad und ihre Schichtdicken sind zur besseren Vergleichbarkeit identisch. Es wird ausschließlich der Dotand in der Emissionsschicht variiert. Beispiel 3 beschreibt einen Vergleichsstandard nach dem Stand der Technik, bei dem die Emissionsschicht aus dem Matrixmaterial CBP und dem Gastmaterial Tris(phenylisochinolin)iridium (Ir(piq)₃) besteht. Des Weiteren wird eine OLED mit einer Emitterschicht bestehend aus dem Matrixmaterial CBP und den Gastmaterialien Ir1 und Ir2 (Beispiele 4 und 5) beschrieben. Analog dem o. g. allgemeinen Verfahren werden OLEDs mit folgendem Aufbau erzeugt:
- PEDOT: 60 nm (aus Wasser aufgeschleudert; PEDOT bezogen von H. C. Starck, Goslar; Poly-[3,4-ethylendioxy-2,5- thiophen]), (HIL)
- NaphDATA: 20 nm (aufgedampft; NaphDATA bezogen von SynTec; 4,4',4"-Tris(N-1-naphthyl)-N-phenyl-amino)triphenyl- amin, (HTL)
- S-TAD: 20 nm (aufgedampft; S-TAD synthetisiert nach WO 99/12888; 2,2',7,7'-Tetrakis(diphenylamino)- spirobifluoren), (HTL)
- Emitter-Schicht:: (EML)
- CPB: 20 nm (aufgedampft; CPB bezogen von ALDRICH und weiter gereinigt, schließlich noch zweimal sublimiert; 4,4'-Bis-(N-carbazolyl)biphenyl)
- **Ir1** und **Ir2**: (10 % Dotierung, aufgedampft; synthetisiert nach Beispiel 1 und 2)
- ODER: Ir(piq)₃: (10 % Dotierung, aufgedampft; synthetisiert nach WO 03/0068526), Vergleichsbeispiel
- BCP: 10 nm (aufgedampft; BCP bezogen von ABCR, verwendet wie erhalten; 2,9-Dimethyl-4,7-diphenyl-1,10- phenanthrolin), (HBL)
- AIQ₃: 10 nm (aufgedampft: AlQ₃ bezogen von SynTec; Tris(chinolinato)aluminium(III)), (ETL)
- LiF: 1 nm
- Al: 100 nm.

Diese noch nicht optimierten OLEDs werden standardmäßig charakterisiert; hierfür werden die Elektrolumineszenzspektren, die Effizienz (gemessen in cd/A) in Abhängigkeit von der Helligkeit, berechnet aus Strom-Spannungs-Helligkeit-Kennlinien (IUL-Kennlinien), und die Lebensdauer bestimmt.

Mit OLEDs hergestellt mit dem Dotanden Ir(piq)₃ erhält man unter den oben beschriebenen Bedingungen typischerweise eine maximale Effizienz von etwa 6.5 cd/A bei Farbkoordinaten von CIE: x = 0.68, y = 0.32. Für die Referenzleuchtdichte von 100 cd/m² werden Spannungen von 6.2 V benötigt. Die Lebensdauer beträgt etwa 250 h bei einer Anfangsleuchtdichte von 500 cd/m² (s. Tabelle 1).

Im Gegensatz dazu zeigen OLEDs hergestellt mit den erfindungsgemäßen Dotanden **Ir1** und **Ir2** maximale Effizienzen von 5.6 bis 5.8 cd/A bei Farbkoordinaten (CIE) von x = 0.70, y = 0.30, wobei die benötigten Spannungen für die Referenzleuchtdichte von 100 cd/m² im Bereich von 5.3 bis 5.4 V liegen (s. Tabelle 1). Die Lebensdauer bei einer Anfangsleuchtdichte von 500 cd/m² ist mit 390 h bis 510 h besser als die des Referenzmaterials Ir(piq)₃ (s. Tabelle 1).

**Tabelle 1: Device-Ergebnisse mit erfindungsgemäßen Dotanden in CBP als Matrix**

| Beispiel | EML | Max. Eff. [cd/A] | Spannung [V] bei 100 cd/m² | CIE (x, y) | Lebensdauer [h] (Anfangs-helligkeit 500 cd/m²) |
|---|---|---|---|---|---|
| Beispiel 3 | CBP: 10% | | | | |
| (Vergleich) | Ir(piq)₃ | 6.5 | 6.2 | 0.68/ | 250 |
| | (30 nm) | | | 0.32 | |
| | CBP : 10% | | | | |
| Beispiel 4 | **Ir1** (30 nm) | 5.8 | 5.4 | 0.70 / 0.30 | 390 |
| | CBP : 10% | | | | |
| Beispiel 5 | **Ir2** (30 nm) | 5.6 | 5.3 | 0.70 / 0.30 | 510 |

### Beispiele 6 bis 8: Weitere Device-Beispiele mit erfindungsgemäßen Dotanden

Die erfindungsgemäßen Dotanden **Ir1** und **Ir2** sowie das Vergleichsmaterial Ir(piq)₃ gemäß dem Stand der Technik werden in OLEDs, enthaltend das Matrixmaterial **M1** gemäß WO 04/093207, getestet. Analog dem in Beispiel 3-5 aufgeführten Verfahren werden OLEDs mit folgendem Aufbau erzeugt:
- PEDOT: 80 nm (aus Wasser aufgeschleudert; PEDOT bezogen von H. C. Starck, Goslar; Poly-[3,4-ethylendioxy-2,5- thiophen]), (HIL)
- NaphDATA: 20 nm (aufgedampft; NaphDATA bezogen von SynTec; 4,4',4"-Tris(N-1-naphthyl)-N-phenyl-amino)triphenyl- amin), (HTL)
- S-TAD: 20 nm (aufgedampft; S-TAD synthetisiert nach WO 99/12888; 2,2',7,7'-Tetrakis(diphenylamino)- spirobifluoren), (HTL)
- Emitter-Schicht:: (EML)
- **M1**: Bis(9,9'-spirobifluoren-2-yl)keton (aufgedampft, synthetisiert nach WO 2004/093207) **Ir1** und **Ir2** (10 % Dotierung, aufgedampft; synthetisiert nach Beispiel 1 und 2)
- ODER: Ir(piq)₃: (10 % Dotierung, aufgedampft; synthetisiert nach WO 03/0068526)
- **HBM1**: 2,7-Bis(4-biphenyl-1-yl)-2`,7`-di-tert-butyl-spiro-9,9`- bifluoren (aufgedampft; synthetisiert nach WO 05/011334)
- AIQ₃: (aufgedampft; AIQ₃ bezogen von SynTec; Tris(chinolinato)aluminium(III)), (ETL);
- Ba-Al: 3 nm Ba, darauf 150 nm Al.

Diese noch nicht optimierten OLEDs werden standardmäßig charakterisiert; hierfür werden die Elektrolumineszenzspektren, die Effizienz (gemessen in cd/A) in Abhängigkeit von der Helligkeit, berechnet aus Strom-Spannungs-Helligkeit-Kennlinien (IUL-Kennlinien), und die Lebensdauer bestimmt. Die Ergebnisse, die mit diesen OLEDs erhalten werden, sind in Tabelle 2 zusammengefasst.

Das Matrixmaterial **M1,** das Lochblockiermaterial **HBM1** und der Vergleichsdotand Ir(piq)₃ sind im Folgenden der Übersichtlichkeit halber abgebildet:

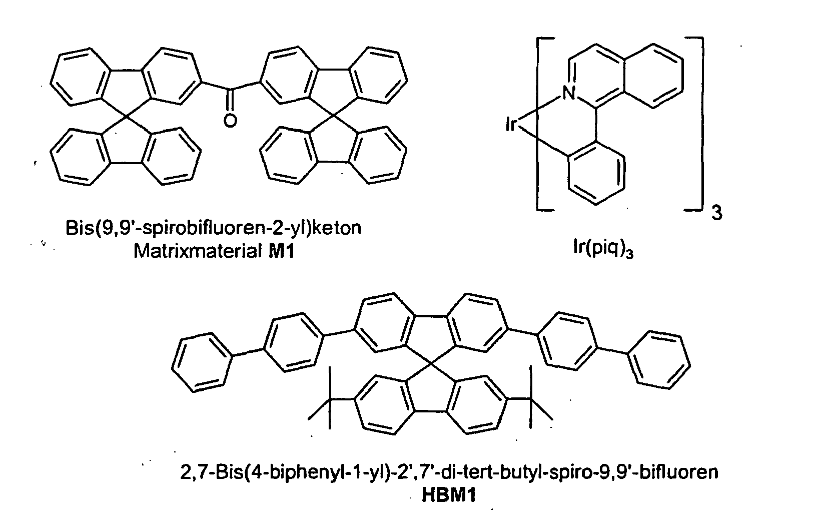

**Tabelle 2: Device-Ergebnisse mit erfindungsgemäßen Dotanden in M1 als Matrix**

| Beispiel | EML | Max. Eff. [cd/A] | Spannung [V] bei 100 cd/m² | CIE (x, y) | Lebensdauer [h] (Anfangshelligkeit 1000 cd/m²) |
|---|---|---|---|---|---|
| Beispiel 6 | **M1**:10% | | | | |
| (Vergleich) | Ir(piq)₃ | 7.4 | 5.8 | 0.68 / 0.32 | 8300 |
| | (30 nm) | | | | |
| | **M1:**10% | | | | |
| Beispiel 7 | **Ir1** | 6.5 | 4.9 | 0.70 / 0.30 | 8500 |
| | (30 nm) | | | | |
| | **M1:**10% | | | | |
| Beispiel 8 | **Ir2** | 5.7 | 4.8 | 0.70 / 0.30 | 9000 |
| | (30 nm) | | | | |

Wie man den Tabellen 1 und 2 entnehmen kann, zeigen die erfindungsgemäßen Emitter tiefrote Emission mit besseren Farbkoordinaten als das Vergleichsmaterial gemäß dem Stand der Technik und eine geringere Betriebsspannung bei verbesserter Lebensdauer.

## Patentansprüche

1. Verbindungen gemäß Formel (1)
M(L)ₙ(L')ₘ(L")ₒ Formel (1)
enthaltend eine Teilstruktur M(L)ₙ gemäß Formel (2), 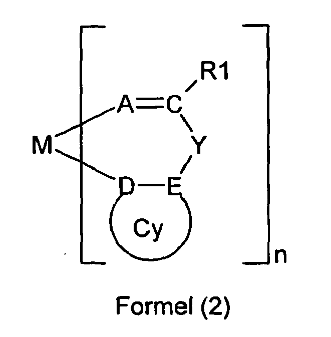 wobei für die verwendeten Symbole und Indizes gilt:
M ist ein Übergangsmetall;
A ist gleich oder verschieden bei jedem Auftreten NR;
D ist ein sp²-hybridisiertes Kohlenstoffatom, das an M bindet;
E ist gleich oder verschieden bei jedem Auftreten ein sp²-hybridisiertes Kohlenstoff oder Stickstoffatom;
Cy ist gleich oder verschieden bei jedem Auftreten ein Homo- oder Heterocyclus, der über ein sp²-hybridisiertes Kohlenstoffatom an M bindet und an den gegebenenfalls eine oder mehrere Gruppen R gebunden sind;
Y ist gleich oder verschieden bei jedem Auftreten CR₂, C(=O), C(=NR), C(=N-NR₂), C(=CR₂), SiR₂, O, S, S(=O), S(=O)₂, Se, NR, PR, P(=O)R, AsR, As(=O)R oder BR;
R, R¹ ist gleich oder verschieden bei jedem Auftreten H, F, Cl, Br, I, CN, B(OH)₂, B(OR²)₂, NO₂, eine geradkettige Alkyl- oder Alkoxy- gruppe mit 1 bis 40 C-Atomen oder eine verzweigte oder cyclische. Alkyl- oder Alkoxygruppe mit 1 bis 40 C-Atomen, wobei jeweils eine oder mehrere nicht benachbarte CH₂-Gruppen durch -R²C=CR²-, -C=C-, Si(R²)₂, Ge(R²)₂, Sn(R²)₂, -O-, -S-, -NR²-,- (C=O)-, -(C=NR²)-, -P=O(R²)- oder -CONR²- ersetzt sein können und wobei ein oder mehrere H-Atome durch F, Cl, Br, I ersetzt sein können, oder ein aromatisches oder heteroaromatisches Ringsystem bzw. eine Aryloxy- oder Heteroaryloxygruppe mit 5 bis 40 C-Atomen, welches durch einen oder mehrere nicht- aromatische Reste R substituiert sein kann; dabei kann R auch mit Cy ein weiteres aliphatisches oder aromatisches Ringsystem bilden; dabei können die Substituenten R, welche an Y binden, mit dem Cyclus Cy und/oder mit dem Rest R1 einen Fünfring oder einen Sechsring bilden;
R² ist gleich oder verschieden bei jedem Auftreten H oder ein aliphatischer oder aromatischer Kohlenwasserstoffrest mit 1 bis 20 C-Atomen, bei dem einzelne H-Atome auch durch F ersetzt sein können; dabei können auch zwei oder mehrere Reste R² miteinander ein Ringsystem bilden;
n ist 1, 2 oder 3;
dabei sind die Liganden L' und L" in Formel (1) monoanionische, zweizähnig chelatisierende Liganden; m und o sind gleich oder verschieden bei jedem Auftreten 0, 1 oder 2; dabei gilt, dass n + m + o = 2 für quadratisch-planar koordinierte Metalle und n + m + o = 3 für oktaedrisch koordinierte Metalle ist.

2. Verbindungen gemäß Anspruch 1, **dadurch gekennzeichnet, dass** Cy ein aromatisches oder heteroaromatisches System darstellt.

3. Verbindungen gemäß Anspruch 1 oder 2 gemäß Formel (1), enthaltend eine Teilstruktur M(L)ₙ gemäß Formel (2a), 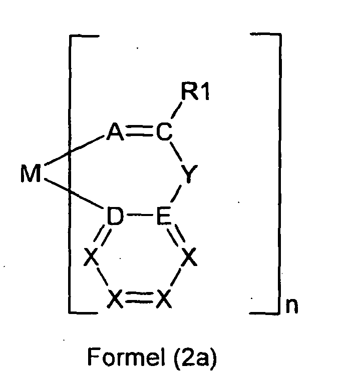 wobei M, A, Y, R, R¹, R², L', L", n, m und o dieselbe Bedeutung haben, wie in Anspruch 1 beschrieben, und für die weiteren Symbole gilt:
D ist ein sp²-hybridisiertes Kohlenstoffatom, das an M bindet;
X ist gleich oder verschieden bei jedem Auftreten CR¹, N oder P; oder
(X-X) bzw. (X=X) (also zwei benachbarte X) steht für NR¹, S oder O; oder
(X-X) bzw. (X=X) (also zwei benachbarte X) steht für CR¹, N oder P, falls das Symbol E im entsprechenden Cyclus für N steht; mit der Maßgabe, dass Cy einen 5- oder 6-Ring darstellt;
E ist gleich oder verschieden bei jedem Auftreten C oder N mit der Maßgabe, dass, falls das Symbol E für N steht, genau eine Einheit X-X (also zwei benachbarte X) in Cy gleich CR¹, N oder P ist.

4. Verbindungen der Formel (1a) gemäß Anspruch 3
M(L)ₙ(L')ₘ(L")ₒ Formel (1a)
enthaltend mindestens eine Teilstruktur M(L)ₙ der Formel (2b), 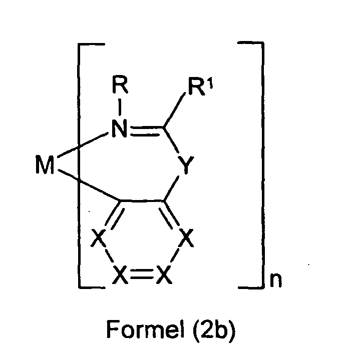 und gegebenenfalls enthaltend eine Teilstruktur M(L')ₘ der Formel (3), 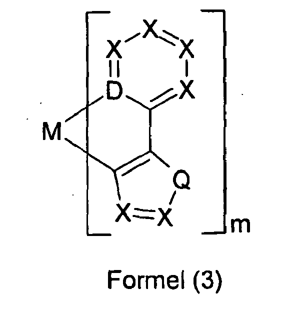 wobei M, A, Y, D, R, R¹, R², L", n, m und o dieselbe Bedeutung haben, wie in Anspruch 1 und 3 beschrieben, und weiterhin gilt:
X ist gleich oder verschieden bei jedem Auftreten CR¹ oder N; oder
(X-X) bzw. (X=X) (also zwei benachbarte X) steht für NR¹, S oder O; mit der Maßgabe, dass Cy einen 5- oder 6-Ring darstellt;
Q ist gleich oder verschieden bei jedem Auftreten -CR¹=CR¹-, -N=CR'-, -N=N-, NR¹, O oder S.

5. Verbindungen gemäß einem oder mehreren der Ansprüche 1 bis 4, enthaltend eine Teilstruktur M(L)ₙ gemäß Formel (2g), Formel (2h) oder Formel (2i), 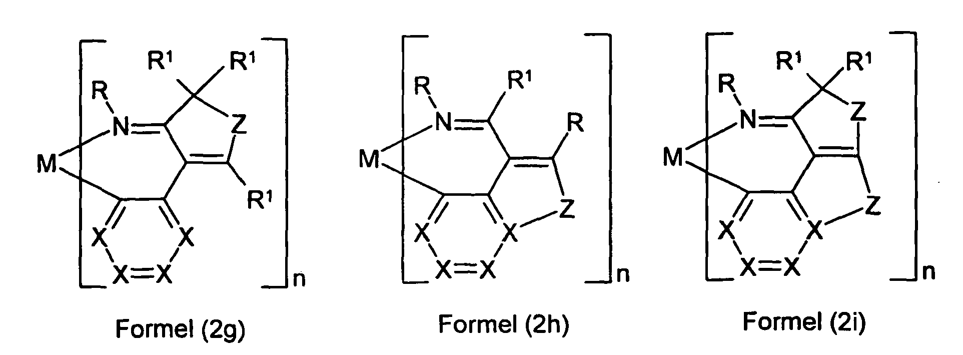 wobei M, X, R, R¹, R² und n dieselbe Bedeutung haben, wie in Anspruch 1 und 3 beschrieben, und für das Symbol Z gilt:
Z ist bei jedem Auftreten gleich oder verschieden eine bivalente Gruppe -C(R¹)₂-, -C(=O)-, -C[=C(R¹⁾₂]-, -C(R¹)₂-C(R¹)₂-, -C(R¹)₂-C(R¹)₂-C(R¹)₂-, -C(R¹)₂-O-C(R¹)₂-, -C(R¹)₂-N(R¹)-, -C(R¹)=C(R¹)-, -C(R¹)=N-, -O-, -S-, -N(R¹)-, -P(R¹)-, -P(=O)(R¹)- oder -B(R¹)-_{.}

6. Verbindungen gemäß einem oder mehreren der Ansprüche 1 bis 5, **dadurch gekennzeichnet, dass** die Liganden L" ausgewählt sind aus der Gruppe der 1,3-Diketonate abgeleitet von 1,3-Diketonen, der 3-Ketonate abgeleitet von 3-Ketoestern, der Carboxylate abgeleitet von Aminocarbonsäuren, der Salicyliminate abgeleitet von Salicyliminen und der Borate stickstoffhaltiger Heterocyclen.

7. Verbindungen gemäß einem oder mehreren der Ansprüche 1 bis 6, **dadurch gekennzeichnet, dass** für das Symbol M = Rh, Ir, Pd oder Pt gilt.

8. Verbindungen gemäß einem oder mehreren der Ansprüche 1 bis 7, **dadurch gekennzeichnet, dass** für den Index n = 2 oder 3 gilt.

9. Verbindungen gemäß einem oder mehreren der Ansprüche 1 bis 8, **dadurch gekennzeichnet, dass** das Symbol Y gleich oder verschieden CR₂, C(=O), C(=NR), C(=CR₂),O, S, NR oder BR ist.

10. Verbindungen gemäß einem oder mehreren der Ansprüche 1 bis 9, **dadurch gekennzeichnet, dass** für das Symbol X = CR¹ gilt.

11. Verbindungen gemäß einem oder mehreren der Ansprüche 1 bis 10, **dadurch gekennzeichnet, dass** sie aus den Strukturen (1) bis (28) ausgewählt sind, die durch R¹ substituiert oder unsubstituiert sind
| | | |
|---|---|---|
| 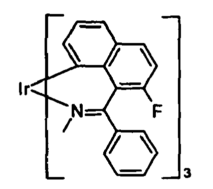 | 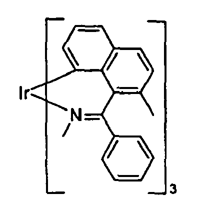 | |
| (1) | (2) | |
| 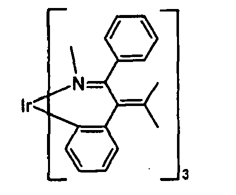 | 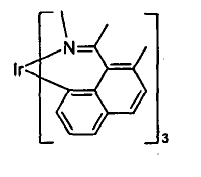 | 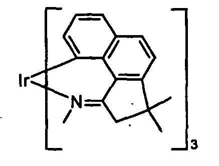 |
| (4) | (5) | (6) |
| 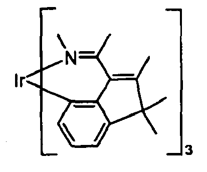 | 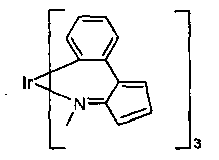 | 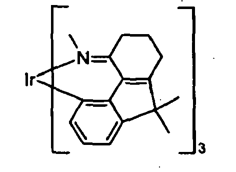 |
| (7) | (8) | (9) |
| 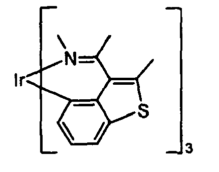 | 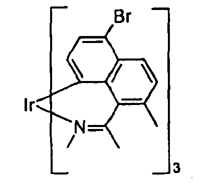 | 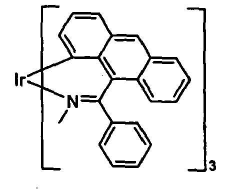 |
| (10) | (11) | (12) |
| 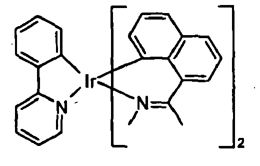 | 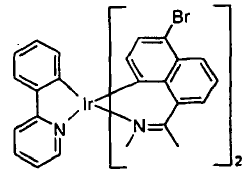 | 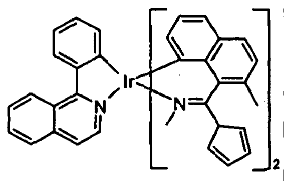 |
| (13) | (14) | (15) |
| 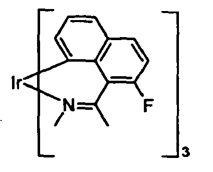 | | |
| (16) | | |
| 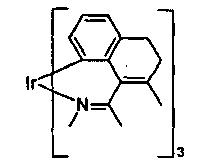 | 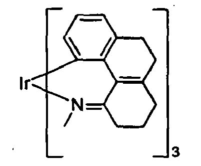 | 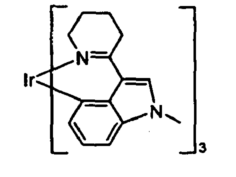 |
| (19) | (20) | (21) |
| 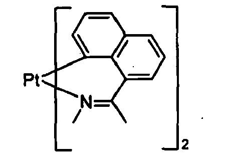 | 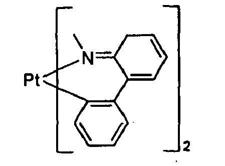 | 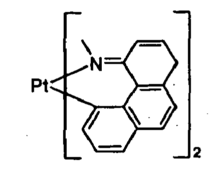 |
| (22) | (23) | (24) |
| 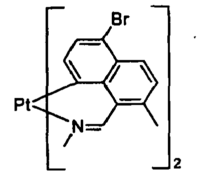 | 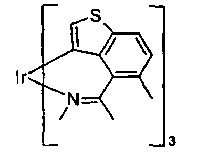 | 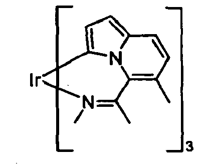 |
| (25) | (26) | (27) |
| 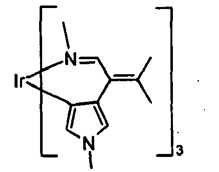 | | |
| (28) | | |

12. Verfahren zur Herstellung von Verbindungen gemäß einem oder mehreren der Ansprüche 1 bis 11 durch Umsetzung der entsprechenden freien Liganden mit Metallalkoholaten der Formel (4), mit Metallketoketonaten der Formel (5) oder ein- oder mehrkernigen Metallhalogeniden der Formeln (6), (7) oder (8), 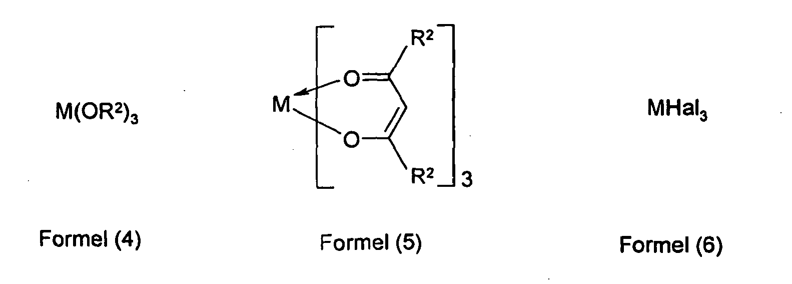 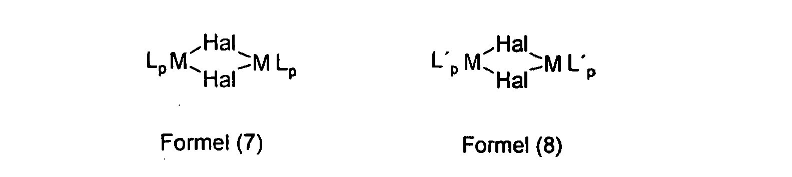 wobei die Symbole M und R² die in Anspruch 1 angegebenen Bedeutungen haben, p = 1 oder 2 und Hal = F, Cl, Br oder I ist, oder mit Metallverbindungen, die sowohl Alkoholat- und/oder Halogenid-und/oder Hydroxy- wie auch Ketoketonatreste tragen.

13. Konjugierte, teilkonjugierte oder nicht-konjugierte Oligomere, Polymere oder Dendrimere, enthaltend eine oder mehrere der Verbindungen gemäß einem oder mehreren der Ansprüche 1 bis 11, wobei mindestens einer der oben definierten Reste R bzw. R¹ eine Bindung zum Polymer oder Dendrimer darstellt.

14. Oligomere, Polymere oder Dendrimere gemäß Anspruch 13, **dadurch gekennzeichnet, dass** weitere Wiederholeinheiten ausgewählt sind aus der Gruppe bestehend aus Fluorenen, Spirobifluorenen, Dihydrophenanthrenen, Indenofluorenen, Phenanthrenen, para-Phenylenen, Carbazolen, Ketonen, Silanen, Triarylaminen oder Thiophenen oder auch mehreren verschiedenen dieser Einheiten.

15. Verwendung von Verbindungen, Oligomeren, Polymeren oder Dendrimeren gemäß einem oder mehreren der Ansprüche 1 bis 11 und/oder 13 bis 14 als aktive Komponente in elektronischen Bauteilen, insbesondere als emittierende Verbindung.

16. Elektronische Bauteile enthaltend eine oder mehrere Verbindungen gemäß einem oder mehreren der Ansprüche 1 bis 11 und/oder Oligomere, Polymere oder Dendrimere gemäß einem oder mehreren der Ansprüche 13 bis 14.

17. Elektronische Bauteile gemäß Anspruch 16, ausgewählt aus der Gruppe der organischen und polymeren Leuchtdioden (OLEDs, PLEDs), organischen Feld-Effekt-Transistoren (O-FETs), organischen Dünnfilmtransistoren (O-TFTs), organischen integrierten Schaltungen (O-ICs), organischen Feld-Quench-Devices (O-FQDs), organischen lichtemittierenden Transistoren (O-LETs), lichtemittierenden elektrochemischen Zellen (LECs), organischen Solarzellen (O-SCs) und organischen Laserdioden (O-Laser).

## Claims

1. Compounds of the formula (1)
M(L)ₙ(L')ₘ(L")ₒ Formula (1)
containing a moiety M(L)ₙ of the formula (2), 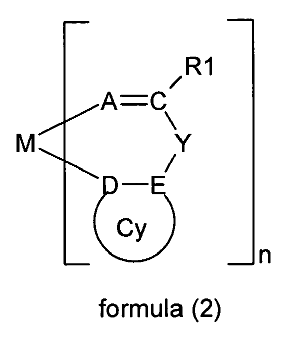 where the following applies to the symbols and indices used:
M is a transition metal;
A is, identically or differently on each occurrence, NR;
D is an sp²-hybridised carbon atom which is bonded to M;
E is, identically or differently on each occurrence, an sp²-hybrid- ised carbon or nitrogen atom;
Cy is, identically or differently on each occurrence, a homo- or het- erocycle which is bonded to M via an sp²-hybridised carbon atom and to which one or more groups R are optionally bonded;
Y is, identically or differently on each occurrence, CR₂, C(=O), C(=NR), C(=N-NR₂), C(=CR₂), SiR₂, O, S, S(=O), S(=O)₂, Se, NR, PR, P(=O)R, AsR, As(=O)R or BR;
R, R¹ are, identically or differently on each occurrence, H, F, Cl, Br, I, CN, B(OH)₂, B(OR²)₂, NO₂, a straight-chain alkyl or alkoxy group having 1 to 40 C atoms or a branched or cyclic alkyl or alkoxy group having 1 to 40 C atoms, where one or more non- adjacent CH₂ groups may each be replaced by -R²C=CR²-, -C=C-, Si(R²)₂, Ge(R²)₂, Sn(R²)_{2,} -O-, -S-, -NR²-, -(C=O)-, -(C=NR²)-, -P=O(R²)- or -CONR²- and where one or more H atoms may be replaced by F, Cl, Br, I, or an aromatic or het- eroaromatic ring system or an aryloxy or heteroaryloxy group having 5 to 40 C atoms, which may be substituted by one or more non-aromatic radicals R; R here may also form a further aliphatic or aromatic ring system with Cy; the substituents R which are bonded to Y may form a five-membered ring or six- membered ring with the ring Cy and/or with the radical R¹;
R² is, identically or differently on each occurrence, H or an aliphatic or aromatic hydrocarbon radical having 1 to 20 C atoms, in which individual H atoms may also be replaced by F; two or more radicals R² here may also form a ring system with one another;
n is 1, 2 or 3;
the ligands L' and L" in formula (1) are monoanionic, bidentate-chelating ligands; m and o are, identically or differently on each occurrence, 0, 1 or 2; n + m + o = 2 here for metals with square-planar coordination and n + m + o = 3 for metals with octahedral coordination.

2. Compounds according to Claim 1, **characterised in that** Cy represents an aromatic or heteroaromatic system.

3. Compounds according to Claim 1 or 2 of the formula (1) containing a moiety M(L)ₙ of the formula (2a), 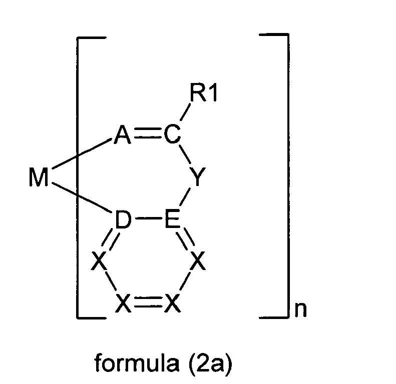 where M, A, Y, R, R¹, R², L', L", n, m and o have the same meaning as described in Claim 1, and the following applies to the other symbols:
D is an sp²-hybridised carbon atom which is bonded to M;
X is, identically or differently on each occurrence, CR¹, N or P; or
(X-X) or (X=X) (i.e. two adjacent X) stands for NR¹, S or O; or
(X-X) or (X=X) (i.e. two adjacent X) stands for CR¹, N or P if the symbol E stands for N in the corresponding ring; with the proviso that Cy represents a 5- or 6-membered ring;
E is, identically or differently on each occurrence, C or N, with the proviso that, if the symbol E stands for N, precisely one unit X-X (i.e. two adjacent X) in Cy is equal to CR¹, N or P.

4. Compounds of the formula (1a) according to Claim 3
M(L)ₙ(L')ₘ(L")ₒ Formula (1a)
containing at least one moiety M(L)ₙ of the formula (2b), 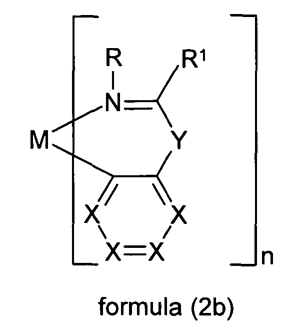 and optionally containing a moiety M(L')ₘ of the formula (3), 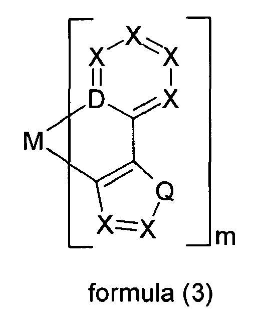 where M, A, Y, D, R, R¹, R², L", n, m and o have the same meaning as described in Claims 1 and 3, and furthermore:
X is, identically or differently on each occurrence, CR¹ or N; or
(X-X) or (X=X) (i.e. two adjacent X) stands for NR¹, S or O; with the proviso that Cy represents a 5- or 6-membered ring;
Q is, identically or differently on each occurrence, -CR¹=CR¹-, -N=CR¹-, -N=N-, NR¹, O or S.

5. Compounds according to one or more of Claims 1 to 4, containing a moiety M(L)ₙ of the formula (2g), formula (2h) or formula (2i), 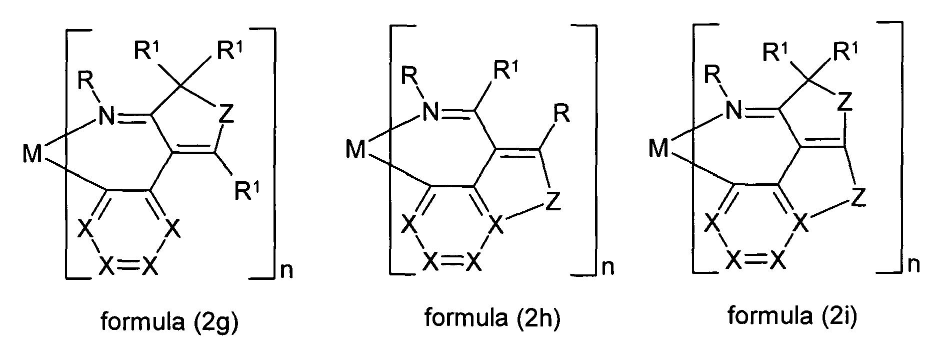 where M, X, R, R¹, R² and n have the same meaning as described in Claims 1 and 3, and the following applies to the symbol Z:
Z is on each occurrence, identically or differently, a divalent group -C(R¹)₂-, -C(=O)-, -C[=C(R¹)₂]-. -C(R¹)₂-C(R¹)₂-, -C(R¹)₂-C(R¹)₂C(R¹)₂-, -C(R¹)₂-O-C(R¹)₂-, -C(R¹)₂-N(R¹)-, -C(R¹)=C(R¹)-, -C(R¹)=N-, -O-, -S-, -N(R¹)-, -P(R¹)-, -P(=O)(R¹)- or -B(R¹)-.

6. Compounds according to one or more of Claims 1 to 5, **characterised in that** the ligands L" are selected from the group of the 1,3-diketon-ates derived from 1,3-diketones, the 3-ketonates derived from 3-ketoesters, the carboxylates derived from aminocarboxylic acids, the salicyliminates derived from salicylimines and the borates of nitrogen-containing heterocycles.

7. Compounds according to one or more of Claims 1 to 6, **characterised in that** the symbol M = Rh, Ir, Pd or Pt.

8. Compounds according to one or more of Claims 1 to 7, **characterised in that** the index n = 2 or 3.

9. Compounds according to one or more of Claims 1 to 8, **characterised in that** the symbol Y is, identically or differently, CR₂, C(=O), C(=NR), C(=CR₂), O, S, NR or BR.

10. Compounds according to one or more of Claims 1 to 9, **characterised in that** the symbol X = CR¹.

11. Compounds according to one or more of Claims 1 to 10, **characterised in that** they are selected from structures (1) to (28), which are substituted by R¹ or are unsubstituted
| | | |
|---|---|---|
| 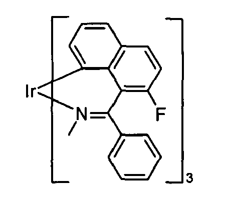 | 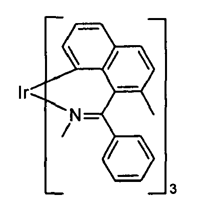 | |
| (1) | (2) | |
| 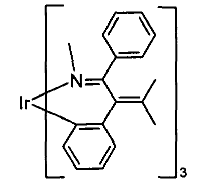 | 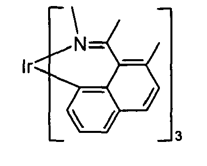 | 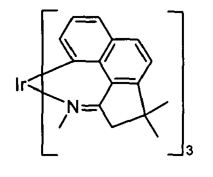 |
| (4) | (5) | (6) |
| 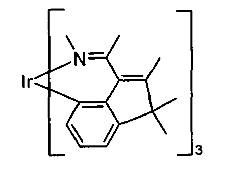 | 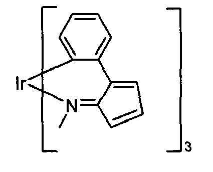 | 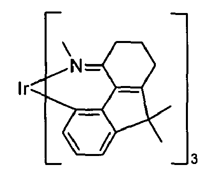 |
| (7) | (8) | (9) |
| 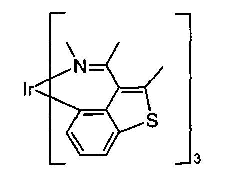 | 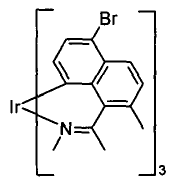 | 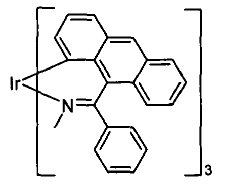 |
| (10) | (11) | (12) |
| 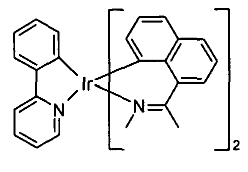 | 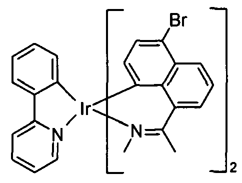 | 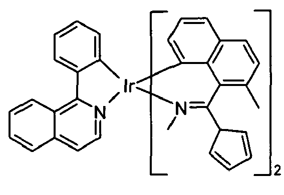 |
| (13) | (14) | (15) |
| 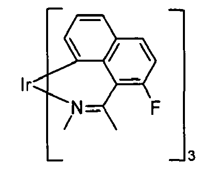 | | |
| (16) | | |
| 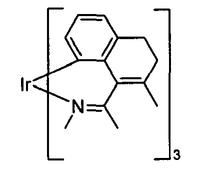 | 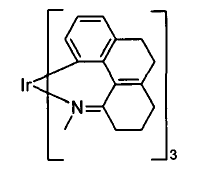 | 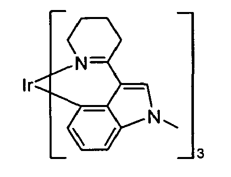 |
| (19) | (20) | (21) |
| 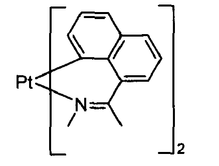 | 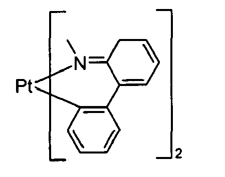 | 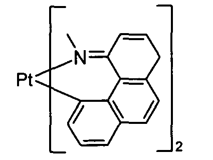 |
| (22) | (23) | (24) |
| 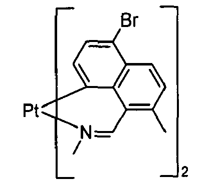 | 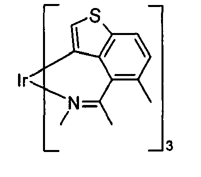 | 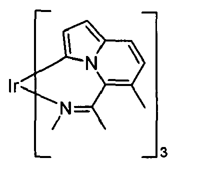 |
| (25) | (26) | (27) |
| 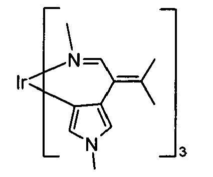 | | |
| (28) | | |

12. Process for the preparation of compounds according to one or more of Claims 1 to 11 by reaction of the corresponding free ligands with metal alkoxides of the formula (4), with metal ketoketonates of the formula (5) or mono- or polycyclic metal halides of the formula (6), (7) or (8), 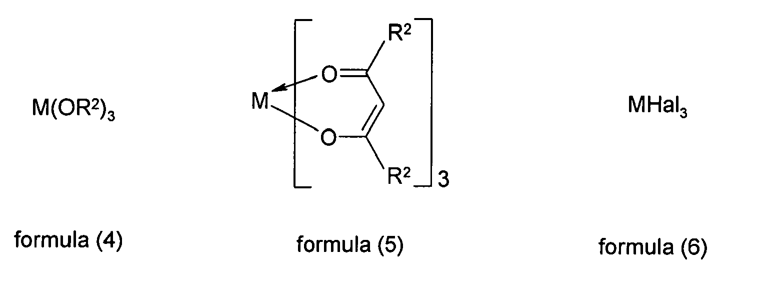 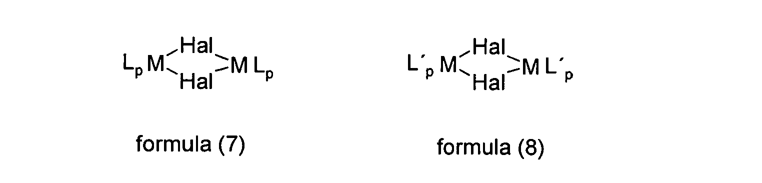 where the symbols M and R² have the meanings indicated in Claim 1, p = 1 or 2 and Hal = F, Cl, Br or I, or with metal compounds which carry both alkoxide and/or halide and/or hydroxyl and also keto-ketonate radicals.

13. Conjugated, partially conjugated or non-conjugated oligomers, polymers or dendrimers comprising one or more of the compounds according to one or more of Claims 1 to 11, where at least one of the radicals R or R¹ defined above represents a bond to the polymer or dendrimer.

14. Oligomers, polymers or dendrimers according to Claim 13, **characterised in that** further recurring units are selected from the group consisting of fluorenes, spirobifluorenes, dihydrophenanthrenes, indeno-fluorenes, phenanthrenes, para-phenylenes, carbazoles, ketones, silanes, triarylamines or thiophenes, or also a plurality of different units thereof.

15. Use of compounds, oligomers, polymers or dendrimers according to one or more of Claims 1 to 11 and/or 13 and 14 as active component in electronic components, in particular as emitting compound.

16. Electronic components comprising one or more compounds according to one or more of Claims 1 to 11 and/or oligomers, polymers or dendrimers according to one or more of Claims 13 and 14.

17. Electronic components according to Claim 16, selected from the group of the organic and polymeric light-emitting diodes (OLEDs, PLEDs), organic field-effect transistors (O-FETs), organic thin-film transistors (O-TFTs), organic integrated circuits (O-ICs), organic field-quench devices (O-FQDs), organic light-emitting transistors (O-LETs), light-emitting electrochemical cells (LECs), organic solar cells (O-SCs) and organic laser diodes (O-lasers).

## Revendications

1. Composés de la formule (1)
M(L)ₙ(L')ₘ(L")ₒ formule (1)
contenant une moitié M(L)ₙ de la formule (2), 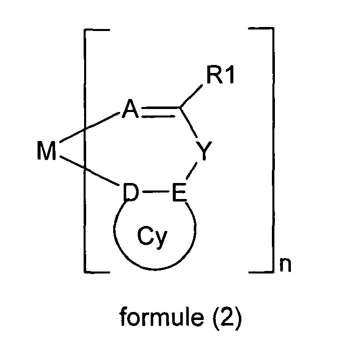 dans laquelle ce qui suit s'applique aux symboles et indices utilisés:
M est un métal de transition;
A est, de façon identique ou différente à chaque occurrence, NR;
D est un atome de carbone sp²-hybridisé qui est lié à M;
E est, de façon identique ou différente à chaque occurrence, un atome de carbone ou d'azote sp²-hybridisé;
Cy est, de façon identique ou différente à chaque occurrence, un homo- ou hétérocycle qui est lié à M via un atome de carbone sp²-hybridisé et auquel un ou plusieurs groupes R sont option- nellement liés;
Y est, de façon identique ou différente à chaque occurrence, CR₂, C(=O), C(=NR), C(=N-NR₂), C(=CR₂), SiR₂, O, S, S(=O), S(=O)₂, Se, NR, PR, P(=O)R, AsR, As(=O)R ou BR;
R, R¹ sont, de façon identique ou différente à chaque occurrence, H, F, Cl, Br, I, CN, B(OH)₂, B(OR²)₂, NO₂, un groupe alkyle ou alcoxy en chaîne droite ayant 1 à 40 atomes de C ou un groupe alkyle ou alcoxy ramifié ou cyclique ayant 1 à 40 atomes de C, dans lequel un ou plusieurs groupes CH₂ non adjacents peuvent chacun être remplacés par -R²C=CR²-, -C=C-, Si(R²)₂, Ge(R²)₂, Sn(R²)₂, -O-, -S-, -NR²-, -(C=O)-, -(C=NR²)-, -P=O(R²)- ou -CONR²- et dans lequel un ou plusieurs atomes de H peuvent être remplacés par F, Cl, Br, I, ou un système de cycle aroma- tique ou hétéroaromatique ou un groupe aryloxy ou hétéroaryl- oxy ayant 5 à 40 atomes de C, qui peut être substitué par un ou plusieurs radicaux non aromatiques R; R ici peut également former un autre système de cycle aliphatique ou aromatique avec Cy; les substituants R qui sont liés à Y peuvent former un cycle à cinq côtés ou un cycle à six côtés avec le cycle Cy et/ou avec le radical R¹;
R² est, de façon identique ou différente à chaque occurrence, H ou un radical hydrocarbone aliphatique ou aromatique ayant 1 à 20 atomes de C, dans lequel des atomes de H individuels peuvent également être remplacés par F; deux radicaux R² ou plus peu- vent ici également former un système de cycle avec un autre;
n est 1, 2 ou 3;
les ligands L' et L" dans la formule (1) sont des ligands chélatants bidentés monoanioniques; m et o sont, de façon identique ou différente à chaque occurrence, 0, 1 ou 2; n + m + o = 2 ici pour des métaux avec une coordination plane carrée et n + m + o = 3 pour des métaux avec une coordination octahédrale.

2. Composés selon la revendication 1, **caractérisés en ce que** Cy représente un système aromatique ou hétéroaromatique.

3. Composés selon la revendication 1 ou 2 de la formule (1) contenant une moitié M(L)ₙ de la formule (2a), 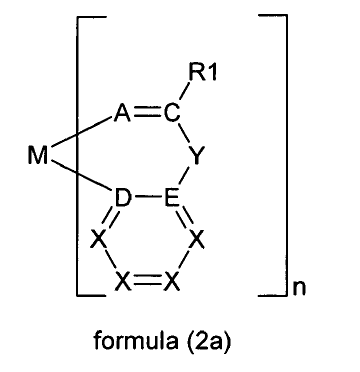 dans laquelle M, A, Y, R, R¹, R², L', L", n, m et o ont les mêmes significations que comme décrit dans la revendication 1, et ce qui suit s'applique aux autres symboles:
D est un atome de carbone sp²-hybridisé qui est lié à M;
X est, de façon identique ou différente à chaque occurrence, CR¹, N ou P; ou
(X-X) ou (X=X) (c'est-à-dire deux X adjacents) représente NR¹, S ou O; ou
(X-X) ou (X=X) (c'est-à-dire deux X adjacents) représente CR¹, N ou P si le symbole E représente N dans le cycle correspondant; sous réserve que Cy représente un cycle à 5 ou 6 côtés;
E est, de façon identique ou différente à chaque occurrence, C ou N, sous réserve que, si le symbole E représente N, précisément une unité X-X (c'est-à-dire deux X adjacents) dans Cy est égale à CR¹, N ou P.

4. Composés de la formule (1 a) selon la revendication 3
M(L)ₙ(L')ₘ(L")ₒ formule (1a)
contenant au moins une moitié M(L)ₙ de la formule (2b), 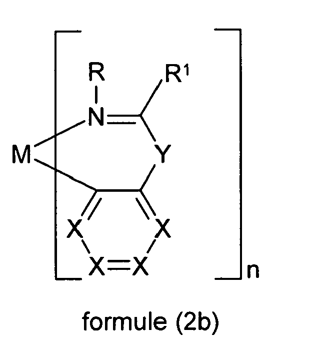 et contenant optionnellement une moitié M(L')ₘ de la formule (3), 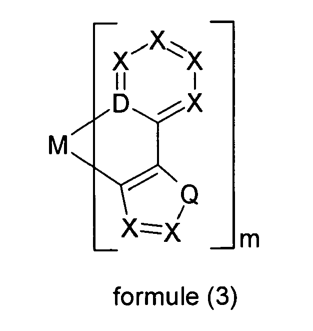 dans laquelle M, A, Y, D, R, R¹, R², L", n, m et o ont la même signification que comme décrit dans la revendication 1 et 3, et en outre:
X est, de façon identique ou différente à chaque occurrence, CR¹ ou N; ou
(X-X) ou (X=X) (c'est-à-dire deux X adjacents) représente NR¹, S ou O; sous réserve que Cy représente un cycle à 5 ou 6 côtés;
Q est, de façon identique ou différente à chaque occurrence, -CR¹=CR¹- -N=CR¹-, -N=N-, NR¹ O ou S.

5. Composés selon une ou plusieurs des revendications 1 à 4, contenant une moitié M(L)ₙ de la formule (2g), formule (2h) ou formule (2i), 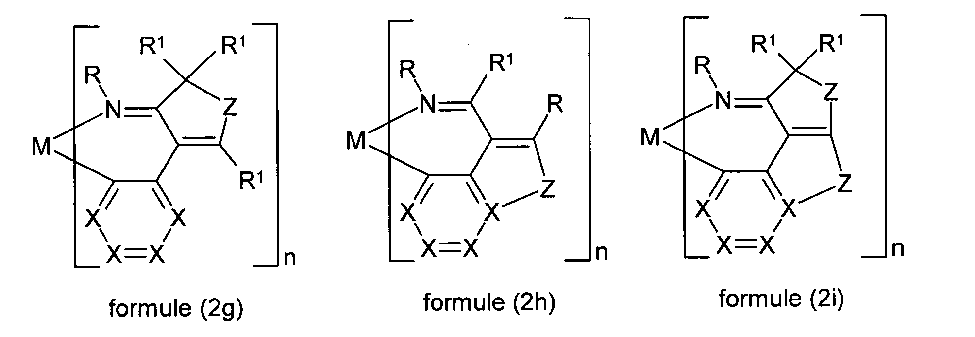 dans lesquelles M, X, R, R¹, R² et n ont la même signification que comme décrit dans la revendication 1 et 3, et ce qui suit s'applique au symbole Z:
Z est, à chaque occurrence de façon identique ou différente, un groupe divalent -C(R¹)₂-, -C(=O)-, -C[=C(R¹)₂]-, -C(R¹)₂-C(R¹)₂-, -C(R¹)₂-C(R¹)₂-C(R¹)₂-, -C(R¹)₂-O-C(R¹)₂-, -C(R¹)₂-N(R¹)-. -C(R¹)=C(R¹)-, -C(R¹)=N-, -O-, -S-, -N(R¹)-, -P(R¹)-, -P(=O)(R¹)- ou -B(R¹)-.

6. Composés selon une ou plusieurs des revendications 1 à 5, **caractérisés en ce que** les ligands L" sont choisis parmi le groupe des 1,3-dicétonates dérivés depuis 1,3-dicétones, des 3-cétonates dérivés depuis 3-cétoesters, des carboxylates dérivés depuis acides amino-carboxyliques, des salicyliminates dérivés depuis salicylimines et des borates d'hétérocycles contenant azote.

7. Composés selon une ou plusieurs des revendications 1 à 6, **caractérisés en ce que** le symbole M = Rh, Ir, Pd ou Pt.

8. Composés selon une ou plusieurs des revendications 1 à 7, **caractérisés en ce que** l'indice n = 2 ou 3.

9. Composés selon une ou plusieurs des revendications 1 à 8, **caractérisés en ce que** le symbole Y est, de façon identique ou différente, CR₂, C(=O), C(=NR), C(=CR₂), O, S, NR ou BR.

10. Composés selon une ou plusieurs des revendications 1 à 9, **caractérisés en ce que** le symbole X = CR¹.

11. Composés selon une ou plusieurs des revendications 1 à 10, **caractérisés en ce qu'**ils sont choisis parmi les structures (1) à (28), qui sont substituées par R¹ ou sont non substituées
| | | |
|---|---|---|
| 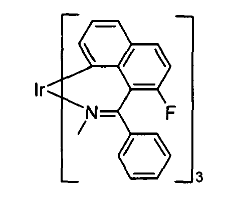 | 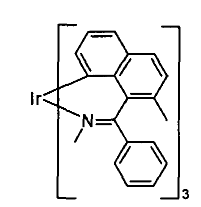 | |
| (1) | (2) | |
| 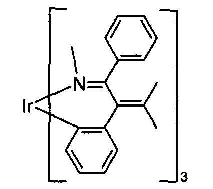 | 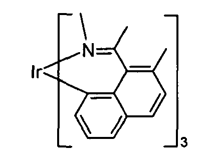 | 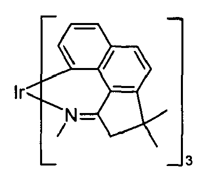 |
| (4) | (5) | (6) |
| 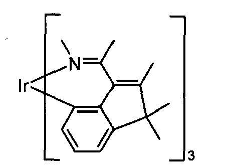 | 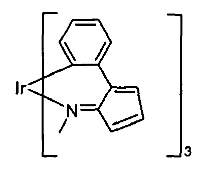 | 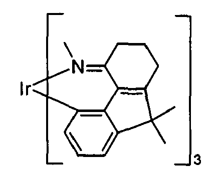 |
| (7) | (8) | (9) |
| 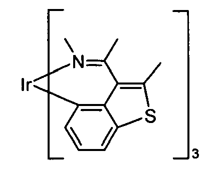 | 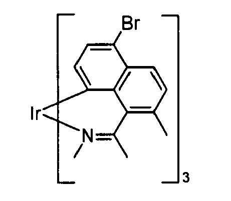 | 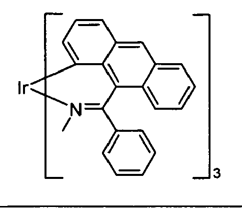 |
| (10) | (11) | (12) |
| 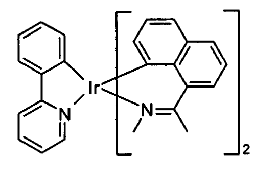 | 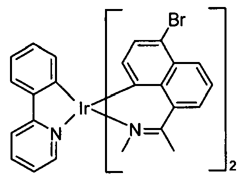 | 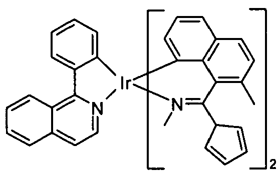 |
| (13) | (14) | (15) |
| 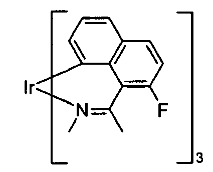 | | |
| (16) | | |
| 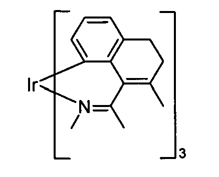 | 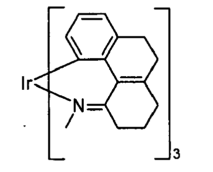 | 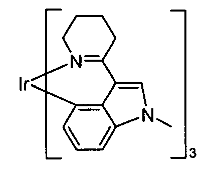 |
| (19) | (20) | (21) |
| 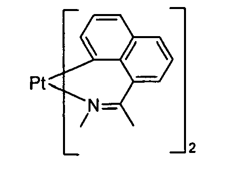 | 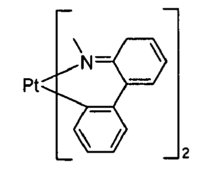 | 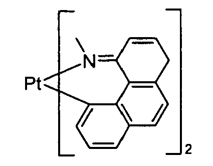 |
| (22) | (23) | (24) |
| 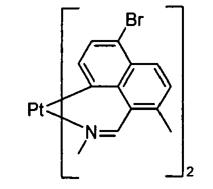 | 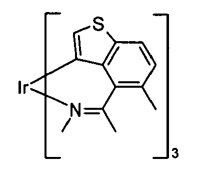 | 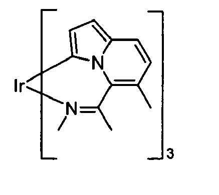 |
| (25) | (26) | (27) |
| 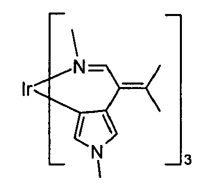 | | |
| (28) | | |

12. Procédé pour la préparation de composés selon une ou plusieurs des revendications 1 à 11 par réaction des ligands libres correspondants avec des alcoxydes de métal de la formule (4), avec des cétocétonates de métal de la formule (5) ou des halogénures de métal monoou polycycliques de la formule (6), (7) ou (8), 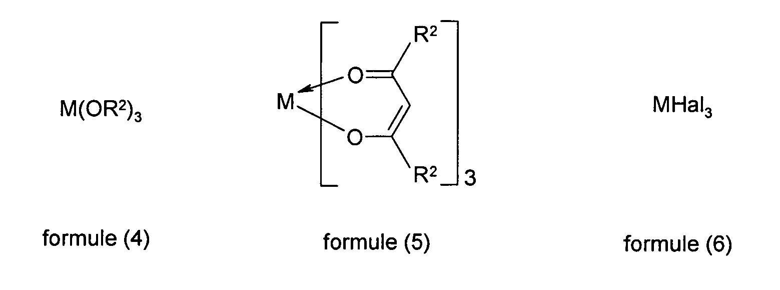 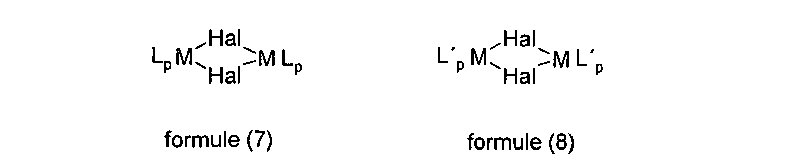 dans laquelle les symboles M et R² ont les significations indiquées dans la revendication 1, p = 1 ou 2 et Hal = F, CI, Br ou l, ou avec des composés de métal qui portent des radicaux à la fois alcoxydes et/ou halogénure et/ou hydroxyle et/ou cétocétonate.

13. Oligomères, polymères ou dendrimères conjugués, partiellement conjugués ou non conjugués comprenant un ou plusieurs composés selon une ou plusieurs des revendications 1 à 11, dans lesquels au moins un des radicaux R ou R¹ définis ci-avant représente une liaison au polymère ou dendrimère.

14. Oligomères, polymères ou dendrimères selon la revendication 13, **caractérisés en ce que** d'autres unités récurrentes sont choisies parmi le groupe constitué de fluorènes, spirobifluorènes, dihydrophénanthrènes, indénofluorènes, phénanthrènes, para-phénylènes, carbazoles, cétones, silanes, triarylamines ou thiophènes, ou également une pluralité de telles unités différentes.

15. Utilisation de composés, oligomères, polymères ou dendrimères selon une ou plusieurs des revendications 1 à 11 et/ou 13 et 14 comme composant actif dans des composants électroniques, en particulier comme composé émetteur.

16. Composants électroniques comprenant un ou plusieurs composés selon une ou plusieurs des revendications 1 à 11 et/ou oligomères, polymères ou dendrimères selon une ou plusieurs des revendications 13 et 14.

17. Composants électroniques selon la revendication 16, choisis parmi le groupe des diodes émettrices de lumière organiques et polymériques (OLED, PLED), transistors à effet de champ organiques (O-FET), transistors à film mince organiques (O-TFT), circuits intégrés organiques (O-IC), dispositifs à pincement de champ organiques (O-FQD), transistors émetteurs de lumière organiques (O-LET), cellules électrochimiques émettrices de lumière (LEC), cellules solaires organiques (O-SC) et diodes laser organiques (O-lasers).
